(19) Europäisches Patentamt
European Patent Office
Office européen des brevets

(11) Publication number: **0 098 141**
**A2**

# (12) EUROPEAN PATENT APPLICATION

(21) Application number: 83303700.5

(22) Date of filing: 27.06.83

(51) Int. Cl.$^3$: C 07 C 177/00
A 61 K 31/557, C 08 B 37/16

(30) Priority: 28.06.82 JP 109806/82

(43) Date of publication of application:
11.01.84 Bulletin 84/2

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Wakatsuka, Hirohisa
3-3-708, Miyano-cho
Takatsuki-shi Osaka(JP)

(72) Inventor: Yamato, Takashi
15-9-3-303, Tsukahara 1-chome
Takatsuki-shi Osaka(JP)

(72) Inventor: Hashimoto, Shinsuke
2-6-905, Shirakawa 3-chome
Ibaraki-shi Osaka(JP)

(74) Representative: Bentham, Stephen et al,
J.A. Kemp & Co. 14 South Square Gray's Inn
London WC1R 5EU(GB)

## (54) Prostaglandin D analogues, process for their preparation and compositions containing them.

(57) Prostaglandin D analogues of the formula:

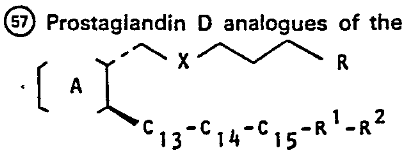

(I)

[wherein [A] represents a group of the formula:

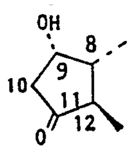

,

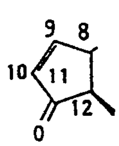

or

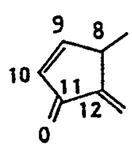

(II) (III) (IV)

X represents ethylene or *cis*-vinylene,
$C_{13}$-$C_{14}$-$C_{15}$ represents
(i) a group of the formula:

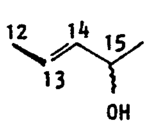

(V)

when [A] represents a group of the formula (II) or (III), or
(ii) a group of the formula

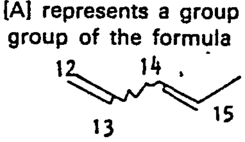

(VI)

when [A] represents a group of the formula (IV), R represents
(i) a group of the formula:

$-COCH_2OH$ or 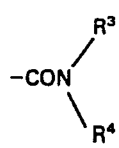

(wherein $R^3$ and $R^4$ are hydrogen or alkyl of 1- 4 carbon atoms) when [A] represents a group of the formula (II), or
(ii) a group of the formula:

$-COCH_2OH$, 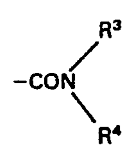 or $-CONHSO_2R^5$

./..

EP 0 098 141 A2

Croydon Printing Company Ltd.

(wherein $R^3$ and $R^4$ are as hereinbefore defined and $R^5$ is alkyl of 1 - 4 carbon atoms) when [A] represents a group of the formula (III) or (IV), $R^1$ represents a single bond or alkylene of 1- 5 carbon atoms, $R^2$ represents alkyl of 1- 8 carbon atoms, cycloalkyl of 4-- 7 carbon atoms either unsubstituted or substituted by alkyl of 1 - 8 carbon atoms, or phenyl or phenoxy optionally substituted by halogen, trifluoromethyl or alkyl or 1 - 4 carbon atoms, the double bond between $C_9$ - $C_{10}$ in formulae (III) and (IV) is Z, the double bond between $C_{13}$ - $C_{14}$ in formula (V) is E, and the double bonds between $C_{12}$ - $c_{13}$ and $C_{14}$ - $C_{15}$ in formula (VI) are E, Z or a mixture thereof, provided that when $R^1$ represents a single bond, $R^2$ does not represent a substituted or unsubstituted phenoxy group] and cyclodextrin clathrates thereof possess anti-tumor activity.

DESCRIPTION

"PROSTAGLANDIN D ANALOGUES, PROCESSES FOR THEIR PREPARATION AND COMPOSITIONS CONTAINING THEM"

This invention relates to new prostaglandin D analogues, to processes for their preparation and pharmaceutical compositions containing them.

Prostaglandins are derivatives of prostanoic acid having the following structure:

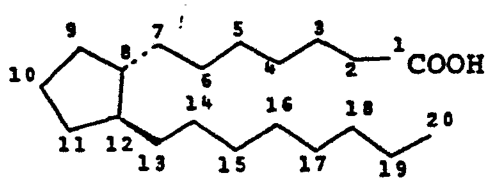

Various types of prostaglandins are known, and their types depend on the structure of the alicyclic ring and the substituents. For example, the alicyclic rings of prostaglandins F (PGF), E (PGE) and D (PGD) have the following structures, respectively:

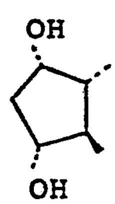

(PGF)

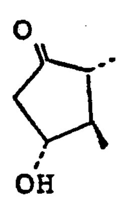

(PGE)

and

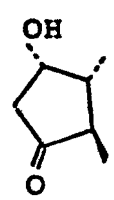

(PGD)

In the above structural formulae or in the other structural formulae in this specification, according to the generally accepted nomenclature, the dotted line indicates that the substituent attached thereto is behind the ring plane, i.e. is of the α-configuration, the bold line indicates that the substituent attached thereto is in front of the ring plane, i.e. is of the β-configuration, and the wavy line ~ indicates that the substituent attached thereto is of the α-configuration or the β-configuration or a mixture thereof.

These compounds are sub-classified according to the positions of the double bonds in the side chains attached to the alicyclic ring at the 8-position and the 12-position. The PG-1 compound has a *trans*-double bond (*trans*- $\Delta^{13}$) between $C_{13}$-$C_{14}$ and the PG-2 compound has a *cis*-double bond between $C_5$-$C_6$ and a *trans*-double bond between $C_{13}$-$C_{14}$ (*cis*- $\Delta^5$, *trans*- $\Delta^{13}$). For example, prostaglandin $D_1$ ($PGD_1$) and prostaglandin $D_2$ ($PGD_2$) may be expressed by the following structural formulae, respectively:

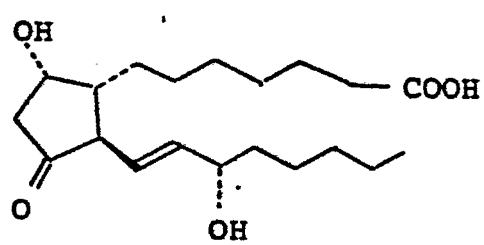

($PGD_1$)

and

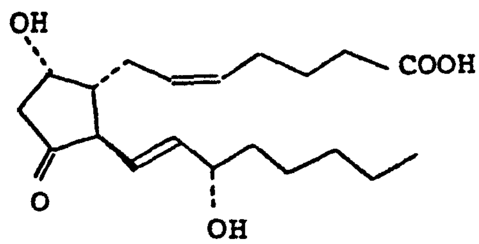

(PGD$_2$)

Further, when one or more methylene groups are removed from the aliphatic group attached at the 12-position of the alicyclic ring of a prostaglandin, said compound is known as a nor-prostaglandin according to the general rule of the organic nomenclature, and the number of the removed methylene groups is indicated by adding di-, tri- etc. before the prefix "nor".

The prostaglandins generally have pharmacological properties. For example, they exert various effects, including the stimulation of contraction of smooth muscles, a hypotensive effect, a diuretic effect, a bronchial dilation effect, the inhibition of lypolysis, the inhibition of platelet aggregation and the inhibition of gastric acid secretion. Therefore, they are useful in treatments of hypertension, thrombosis, asthma and gastric and intestinal ulcers, in the induction of labour and abortion in pregnant mammals, in the prevention of arteriosclerosis and also as diuretics. They are liposoluble substances present in extremely small quantities in the tissues which secrete prostaglandins in vivo in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been discovered that (a) certain novel PGD analogues in which the carboxyl group attached at the 1-position of PGD and PGD analogues is replaced by a glycoloyl group (i.e. $-COCH_2OH$ group) or by certain substituted or unsubstituted amide groups defined hereinafter, (b) further novel compounds in which the hydroxyl group attached at the 9-position of the compounds wherein the carboxyl group is replaced by the glycoloyl group, by the amide group or by an alkylsulfonylamide group as hereinafter defined, has been removed to introduce a cis double bond between $C_9$-$C_{10}$, and (c) further novel compounds in which the hydroxyl group attached at the 15-position of the compounds (b) has been removed to introduce double bonds between $C_{12}$-$C_{13}$ and between $C_{14}$-$C_{15}$ have a surprisingly strong anti-tumour effect whereas they have no or only very weak pharmacological properties possessed by the "natural" prostaglandins.

Heretofore, there have been filed several patent applications relating to PGD analogues. For example, concerning compounds in which the carboxylic acid moiety attached at the 1-position of $PGD_1$ and $PGD_2$ has been converted to other groupings, there are methanesulfonylamide forms [see United States Patent No. 4346228 (Derwent No. 43349Y)], alcohol forms [see United States Patent Nos. 4028419 (Derwent No. 43349Y), 4055602 (Derwent No. 48794Y) and 4032576 (Derwent No. 48793Y)], amine forms [see British Patent Nos. 1554041 and 1554042 (Derwent No. 46957Y)] and ketone forms [see United States Patent No. 4123463 (Derwent No. 81996A)]. However, neither PGD compounds in which the carboxyl group is replaced by the glycoloyl group or an amide group as encomposed by the present invention nor the novel effect of the compounds of the present invention, i.e. their anti-tumour effect has been described heretofore. The above-mentioned patent specifications describe only the pharmacological properties known with the natural prostaglandins and none of the applications refers to an anti-tumour effect.

There have also been several patent applications relating to the derivatives in which the hydroxyl group at the 9-position has been dehydrated, which is a further structural feature in the compounds of the present invention (9-deoxy- $\Delta^9$ derivatives). For example, among the above-mentioned patent specifications, the corresponding 9-deoxy- $\Delta^9$ derivatives are described in all the specifications except United States Patent No. 4346228 (Derwent No. 81216X). However, there is no disclosure of the 9-deoxy- $\Delta^9$ derivatives as encompassed by the present invention nor, as already indicated, any disclosure of anti-tumor activity.

PGD compounds in which the hydroxyl groups at the 9-position and the 15-position have been removed to introduce double bonds $C_9$-$C_{10}$, $C_{12}$-$C_{13}$ and $C_{14}$-$C_{15}$, a further structural feature in the compounds of the present invention, have not hitherto been published.

The present invention accordingly provides PGD analogues of the general formula:

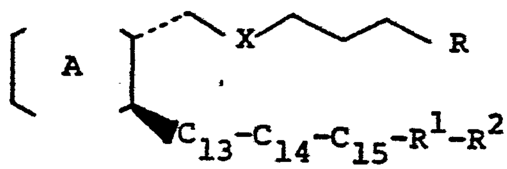

(I)

[wherein [A] represents a group of the formula:

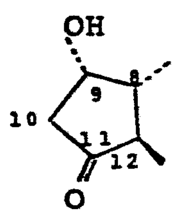

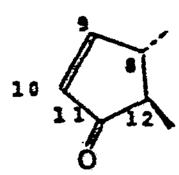

or

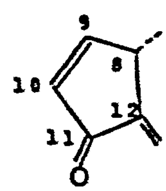

(II) (III) (IV)

X represents an ethylene group ($-CH_2CH_2-$) or a <u>cis</u>-vinylene group ($_{H}\!>C = C<_{H}$), $C_{13}$-$C_{14}$-$C_{15}$ represents

(i) a group of the formula:

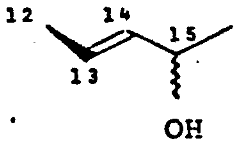

(V)

when [A] represents a group of the formula (II) or (III), or (ii) a group of the formula:

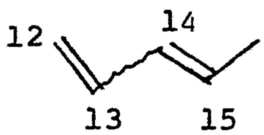

(VI)

when [A] represents a group of the formula (IV), R represents (i) a group of the formula:

$-COCH_2OH$ or 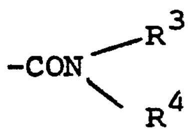

(wherein $R^3$ and $R^4$, which may be the same or different, each represent a hydrogen atom or a straight-chain or branched-chain alkyl group of 1-4 carbon atoms) when [A] represents a group of the formula (II), or (ii) a group of the formula:

$-COCH_2OH$, 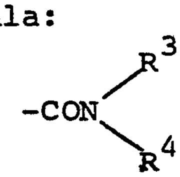 or $-CONHSO_2R^5$

wherein $R^3$ and $R^4$ are as hereinbefore defined and $R^5$ represents a straight-chain or branched-chain alkyl group of 1-4 carbon atoms) when [A] represents a group of the formula (III) or (IV), $R^1$ represents a single bond or a straight-chain or branched-chain alkylene group of 1-5 carbon atoms, $R^2$ represents a straight-chain or branched-chain alkyl group of 1-8 carbon atoms, a cycloalkyl group

of 4-7 carbon atoms either unsubstituted or substituted by at least one straight-chain or branched-chain alkyl group of 1-8 carbon atoms, or a phenyl or phenoxy group either unsubstituted or substituted by at least one halogen atom, trifluoromethyl group or straight-chain or branched-chain alkyl group of 1-4 carbon atoms, the double bond between $C_9$-$C_{10}$ in formulae (III) and (IV) is Z, the double bond between $C_{13}$-$C_{14}$ in formula (V) is E, the double bonds between $C_{12}$-$C_{13}$ and between $C_{14}$-$C_{15}$ in formula VI, which may be in the same or different configurations are E, Z or a mixture thereof (i.e. EZ), with the proviso that when $R^1$ represents a single bond, $R^2$ does not represent a substituted or unsubstituted phenoxy group], and cyclodextrin clathrates thereof.

The compounds of the present invention include 2-decarboxy-2-glycoloyl-PGD derivatives or PGD amide derivatives represented by the general formula:

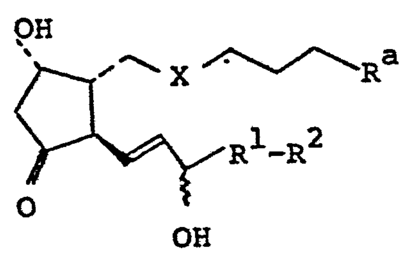

(Ia)

[wherein $R^a$ represents a group of the formula $-COCH_2OH$ or $-CON\begin{smallmatrix}R^3\\R^4\end{smallmatrix}$ (wherein $R^3$ and $R^4$ are as hereinbefore defined) and the other symbols are as hereinbefore defined]; 2-decarboxy-2-glycoloyl-9-deoxy-$\Delta^9$-PGD derivatives, 9-deoxy-$\Delta^9$-

PGD amide derivatives and 9-deoxy-$\Delta^9$-PGD alkylsulfonylamide derivatives represented by the general formula:

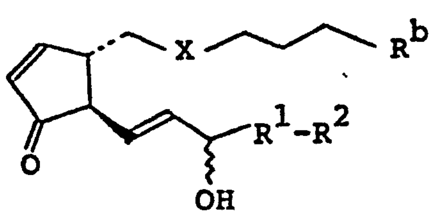

(Ib)

[wherein $R^b$ represents a group of the formula $-COCH_2OH$, 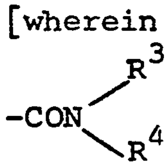 or $-CONHSO_2R^5$ (wherein $R^3$, $R^4$ and $R^5$ are as hereinbefore defined) and the other symbols are as hereinbefore defined]; and further compounds represented by the general formula:

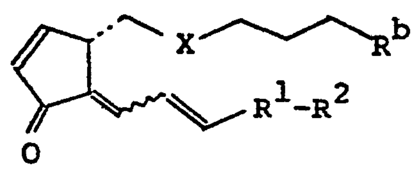

(Ic)

(wherein the various symbols are as hereinbefore defined).

In the compounds of general formula (I), there are several asymmetric carbon atoms. For example, in the compounds of general formula (Ia), there are four asymmetric carbon atoms (at the 8-, 9-, 12- and 15-positions), in the compounds of general formula (Ib), the carbon atoms at the 8-, 12- and 15-positions are asymmetric, and in the compounds of general formula (Ic), the carbon atom at the 8-position is asymmetric. Further asymmetric carbon atoms

may occur in the groups represented by $R^1$, $R^2$, $R^3$, $R^4$ and $R^5$. The presence of asymmetric carbon atoms leads to the existence of isomerism. It is to be understood that all isomers and mixtures thereof arising from the presence of asymmetric carbon atoms are to be considered within the scope of formula (I).

In the general formula (I), the alkylene groups of 1-5 carbon atoms represented by $R^1$ include methylene, ethylene, trimethylene, tetramethylene and pentamethylene groups and isomers thereof; $R^1$ preferably represents a single bond or a methylene or ethylene group.

In the general formula (I), the alkyl groups of 1-8 carbon atoms represented by $R^2$ include methyl, ethyl, propyl, butyl, pentyl, hexyl, heptyl and octyl groups and isomers thereof; butyl, pentyl or hexyl groups either unsubstituted or substituted by one or two methyl or ethyl groups are preferred; pentyl and hexyl groups unsubstituted or substituted by one or two methyl groups are especially preferred.

In the general formula (I), the substituted or unsubstituted cycloalkyl groups represented by $R^2$ include cyclobutyl, cyclopentyl, cyclohexyl and cycloheptyl groups, and cyclobutyl, cyclopentyl, cyclohexyl and cycloheptyl groups substituted by one or more methyl, ethyl, propyl, butyl, pentyl, hexyl, heptyl or octyl groups; cyclobutyl, cyclopentyl or cyclohexyl groups either unsubstituted or substituted by one methyl, ethyl, propyl or butyl group

are preferred; 3-propylcyclopentyl is especially preferred.

In the general formula (I), the substituted or unsubstituted phenyl or phenoxy groups represented by $R^2$ include phenyl and phenoxy groups, and phenyl and phenoxy groups substituted by one or more atoms or groups selected from the fluorine and chlorine atoms and trifluoromethyl, methyl, ethyl, propyl or butyl groups; phenyl or phenoxy groups either unsubstituted or substituted by one chlorine atom, trifluoromethyl group, methyl or ethyl group are preferred; chlorine is a preferred substituent.

In the general formula (I), preferred groupings $R^1$-$R^2$ are butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, 4-butylcyclohexyl, benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl; pentyl, 2-methylhexyl, 3-propylcyclopentyl, benzyl and 3-chlorophenoxymethyl are especially preferred.

In general formula (I), the alkyl groups of 1-4 carbon atoms represented by $R^3$, $R^4$ and $R^5$ include methyl, ethyl, propyl and butyl groups, and isomers thereof; $R^3$ and $R^4$ preferably represent a hydrogen atom or a methyl group, and $R^5$ preferably represents a methyl group.

In general formula (I), X is preferably a cis-vinylene group, and in general formula (I), where $C_{13}$-$C_{14}$-$C_{15}$ represents a group of formula (V), the preferred configuration of the hydroxyl group attached to the carbon atom at the 15-position is the α-configuration.

According to a feature of the present invention, the prostaglandin D analogues of general formula (Ib) wherein the various symbols are as hereinbefore defined may be prepared by subjecting a compound of the general formula:

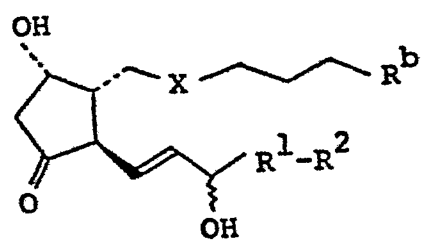

(VII)

(wherein the various symbols are as hereinbefore defined) to a mild dehydration reaction, for example, in a Tris-hydrochloric acid buffer solution at a temperature of 30-40°C.

According to a further feature of the invention prostaglandin D analogues of general formula (Ic) wherein the various symbols are as hereinbefore defined can be prepared by reacting a compound of the general formula (VII) with an aqueous acid, preferably in an inert organic solvent, for example, methylene chloride, chloroform, carbon tetrachloride, diethyl ether, N,N-dimethylformamide, tetrahydrofuran or a mixture of two or more such solvents using as the aqueous acid solution, for example, an aqueous solution of an inorganic acid such as hydrochloric acid, hydrobromic acid, sulfuric acid or phosphoric acid or an organic acid such as acetic acid, propionic acid or oxalic acid, at the reflux temperature of the reaction mixture. The reaction is preferably carried out in tetrahydrofuran using 1 N hydrochloric acid at the reflux temperature of the reaction mixture.

The prostaglandin D analogues of general formula (Ia) wherein the various symbols are as hereinbefore defined are within the scope of general formula (VII).

The compounds of general formula (VII) can be prepared by the sequence of reaction steps illustrated by the following scheme A. In the scheme, $R^6$ represents a tetrahydropyran-2-yl group or tetrahydrofuran-2-yl group either unsubstituted or substituted by at least one alkyl group, or a 1-ethoxyethyl group, preferably a tetrahydropyran-2-yl group, $R^7$ represents a straight-chain or branched-chain acyl group of 2-5 carbon atoms or a benzoyl

group, preferably a benzoyl group, $R^8$ represents a straight-chain or branched-chain alkyl group of 1-4 carbon atoms, preferably a methyl group, and the other symbols are as hereinbefore defined.

The compounds of the general formula (VII) are illustrated as the general formulae (VIIa), (VIIb) and (VIIc) in the scheme A. Further, the compounds of the general formulae (VIIa) and (VIIb) correspond to the compounds of the general formula (Ia). The preparation of compounds of general formula (Ia) by the hydrolysis under acidic conditions of a compound of general formula (XI) or (XV) depicted in scheme A, wherein the various symbols are as hereinbefore defined, constitutes a further feature of the present invention.

By the expression "known methods" as used in this specification, for example to describe some of the reactions depicted in scheme A, is meant methods heretofore used or described in the literature.

Scheme A

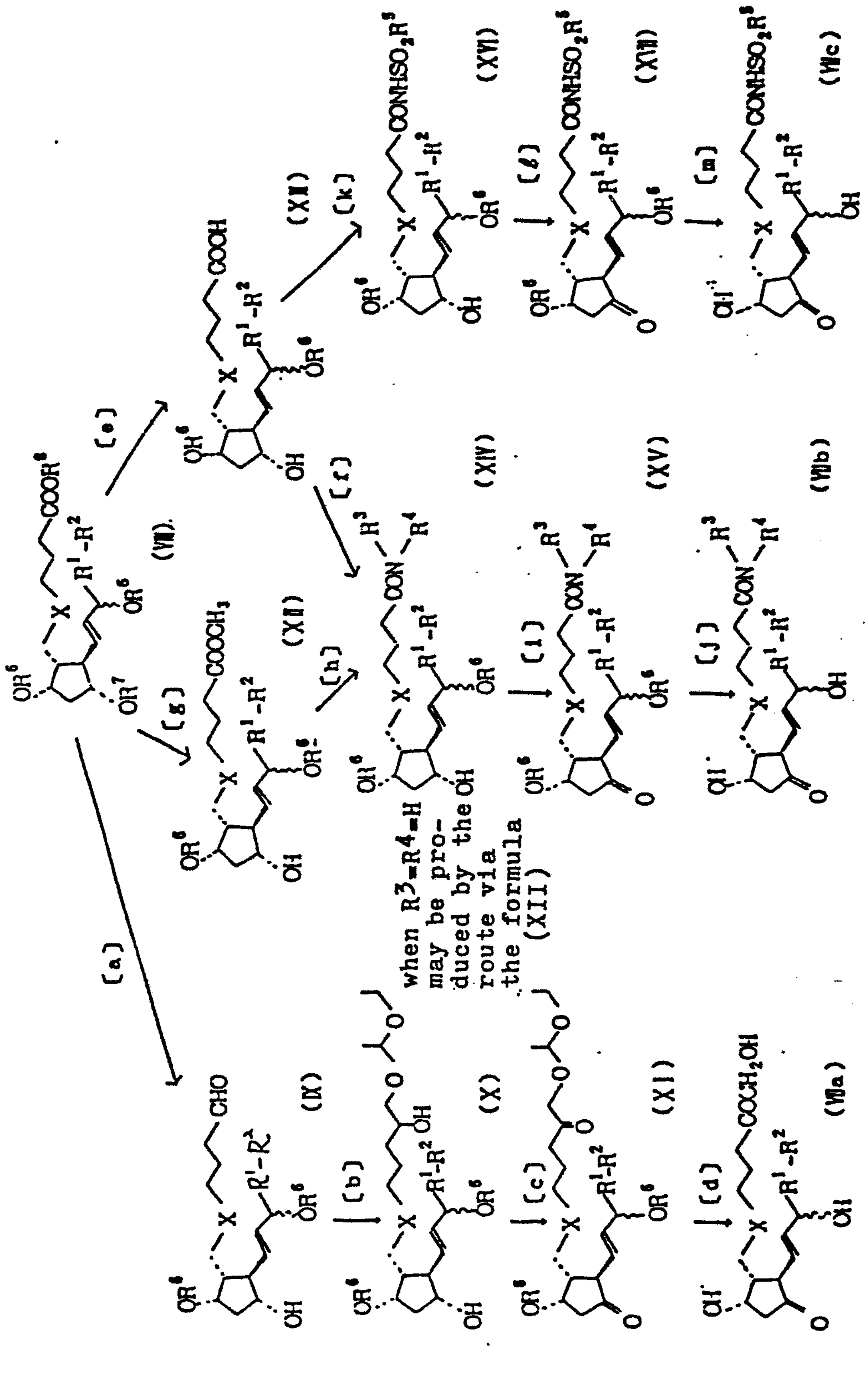
(VII)
(a)
(IX)
(b)
(X)
(c)
(XI)
(d)
(VIIa)
(g)
(XII)
(h)
when $R^3=R^4=H$ may be produced by the route via the formula (XII)
(i)
(j)
(VIIb)
(e)
(XIII)
(f)
(XIV)
(XV)
(k)
(XVI)
(ℓ)
(XVII)
(m)
(VIIc)

The respective steps of the scheme A are now described. The step [a] is conducted by subjecting the compound of formula (VIII) to reduction to convert the carboxylic acid ester group ($COOR^8$) to a group -CHO. This reduction reaction may be conducted, for example, by using diisobutylaluminium hydride (DIBAL) in hexane, tetrahydrofuran or toluene at a temperature of 0°C to -80°C, preferably by using DIBAL in toluene at a temperature of -78°C.

The step [b] can be conducted by known methods to add a 2,4-dioxa-3-methylhexyl group to the compound of the general formula (IX). This reaction can be effected in an inert organic solvent, for example, an ether such as diethyl ether, tetrahydrofuran and 1,2-dimethoxyethane, hydrocarbons such as n-hexane and n-heptane, or a mixture of such solvents, at a temperature of 0°C to -80°C using n-butyl lithium and (2,4-dioxa-3-methylhexyl)-tri-n-butylstannane. Preferably, it is effected by adding dropwise a solution of n-butyl lithium in n-hexane to (2,4-dioxa-3-methylhexyl)-tri-n-butylstannane in tetrahydrofuran at -78°C, and adding dropwise a solution of the aldehyde compound of the general formula (IX) in tetrahydrofuran to the resulting reaction mixture [see J. Amer. Chem. Soc., 100, 1481 (1978)].

The step [c] is an oxidation reaction to convert the hydroxyl group to an oxo group. Such an oxidation reaction is well known, and is described in detail in,

for example,

(a) "Organic Synthetic Chemistry III, Synthesis Part 1", edited by Tetsuji Kameya, published by Nanko-do, page 176-206 (August 1, 1976), or

(b) "Compendium of Organic Synthetic Methods" published by John Wiley & Sons, Inc. (U.S.A.), Vol. 1 (1971), Vol. 2 (1974) and Vol. 3 (1977), Section 48 or 168.

An especially preferred oxidation under mild neutral conditions may be conducted by using, for example, dimethylsulfide - N-chlorosuccinimide complex, thioanisole - N-chlorosuccinimide complex, dimethylsulfide - chlorine complex, thioanisole - chlorine complex [see J. Amer. Chem. Soc., 94, 7586 (1972) with respect to these complexes], dicyclohexylcarbodiimide - dimethylsulfoxide complex [see J. Amer. Chem. Soc., 87, 5661 (1976)], pyridinium chlorochromate ($C_5H_5NHCrO_3Cl$) [see Tetrahedron Letters, 2647 (1975)], sulfuric anhydride - pyridine complex [see J. Amer. Soc., 89, 5505 (1976)], chromyl chloride [see J. Amer. Chem. Soc., 97, 5929 (1975)], chromium trioxide - pyridine complex (for example Collins' reagent), Jones' reagent or chromic acid solution (prepared from chromium trioxide, manganese sulfate, sulfuric acid and water), or oxalyl chloride and dimethylsulfoxide (i.e. Swern oxidation); suitably the oxidation is carried out using Collins' reagent, Jones' reagent or the Swern oxidation. The oxidation reaction using Collins' reagent may be conducted in a halogenated hydrocarbon such as chloroform, methylene

chloride or carbon tetrachloride at a temperature of from room temperature to 0°C, preferably at 0°C, and the oxidation reaction using Jones' reagent can be conducted generally at a temperature of room temperature or below. The Swern oxidation may be conducted by reaction in a halogenated hydrocarbon such as chloroform or methylene chloride at a temperature of from -50°C to -60°C, and then treatment with triethylamine.

The step (d) can be conducted by hydrolysis under acidic conditions. This reaction can be conducted

(1) in an aqueous solution of an organic acid such as acetic acid, propionic acid, oxalic acid or p-toluenesulfonic acid or an aqueous solution of an inorganic acid such as hydrochloric acid, sulfuric acid or phosphoric acid, suitably in the presence of a water-miscible organic solvent, for example, a lower alkanol such as methanol or ethanol (preferably methanol) or an ether such as 1,2-dimethoxyethane, dioxane or tetrahydrofuran (preferably tetrahydrofuran) at a temperature of from room temperature to 75°C (preferably at a temperature of not higher than 45°C), or

(2) in an anhydrous lower alkanol such as methanol or ethanol in the presence of an organic acid such as p-toluenesulfonic acid or trifluoroacetic acid at a temperature of 10-45°C.

The hydrolysis is preferably conducted using a mixture of dilute hydrochloric acid and tetrahydrofuran, a mixture of dilute hydrochloric acid and methanol,

a mixture of acetic acid, water and tetrahydrofuran, a mixture of phosphoric acid, water and tetrahydrofuran, or a mixture of p-toluenesulfonic acid and anhydrous methanol.

The step (e) can be conducted in an aqueous alkanol such as aqueous methanol or aqueous ethanol using a hydroxide of an alkali metal such as sodium or potassium at a temperature of not lower than room temperature, preferably 50-60°C.

The step (f) can be conducted by synthesizing an acid halide or acid anhydride of the compound of the general formula (XIII) and reacting the resulting compound with a compound of the general formula:

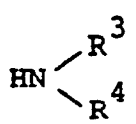

(XVIII)

(wherein $R^3$ and $R^4$ are as hereinbefore defined) in an inert organic solvent, for example, methylene chloride, tetrahydrofuran or N,N-dimethylformamide, at from 0°C to room temperature. Suitably, it is conducted by reaction in methylene chloride in the presence of a base such as triethylamine using isobutyl chloroformate or trimethylacetyl chloride at 0°C to make an acid anhydride of the compound of the general formula (XIII), and then reaction of the acid anhydride with a compound of the general formula (XVIII) at room temperature.

The compound of the general formula (XIV) wherein $R^3$ and $R^4$ both represent a hydrogen atom can also be synthesized by the route via the steps [g] and [h].

The step [g] can be conducted by reaction in methanol using a carbonate of an alkali metal such as sodium or potassium at a temperature of not lower than room temperature, preferably at 50-60°C.

The step [h] can be conducted by reaction in a lower alkanol such as methanol or ethanol in the presence of ammonium chloride using concentrated aqueous ammonia at 50-60°C.

The steps [i] and [j] can be conducted as hereinbefore described for steps [c] and [d] respectively.

The step [k] can be conducted by synthesizing an acid halide or acid anhydride of the compound of the general formula (XIII) and reacting the resulting compound with a compound of the general formula:

$$MNHSO_2R^5 \qquad (XIX)$$

(wherein M represents an alkali metal, preferably sodium, and $R^5$ is as hereinbefore defined) in an inert organic solvent, for example, methylene chloride, tetrahydrofuran, N,N-dimethylformamide, hexamethylphosphoramide, dimethylsulfoxide or a mixture thereof, at from 0°C to room temperature. Suitable, it is conducted by reaction in N,N-dimethylformamide in the presence of a base such as triethylamine using isobutyl chloroformate or trimethylacetyl

chloride at 0°C to make an acid anhydride of the compound of the general formula (XIII), and subsequently reacting the acid anhydride with a sodium derivative of the general formula (XIX) in suspension in dimethylsulfoxide in the presence of hexamethylphosphoramide at a temperature of about 50°C.

The steps [l] and [m] can be conducted as hereinbefore described for steps [c] and [d] respectively.

In the above-described scheme A, the compound of the general formula (VIII) employed as the starting material can be produced by the sequence of the reaction steps illustrated by the following scheme B. In the scheme, $R^9$ represents a straight-chain or branched-chain alkyl group of 1-4 carbon atoms or a phenyl group, preferably a n-butyl group or a phenyl group, and the other symbols are as hereinbefore defined.

Scheme B

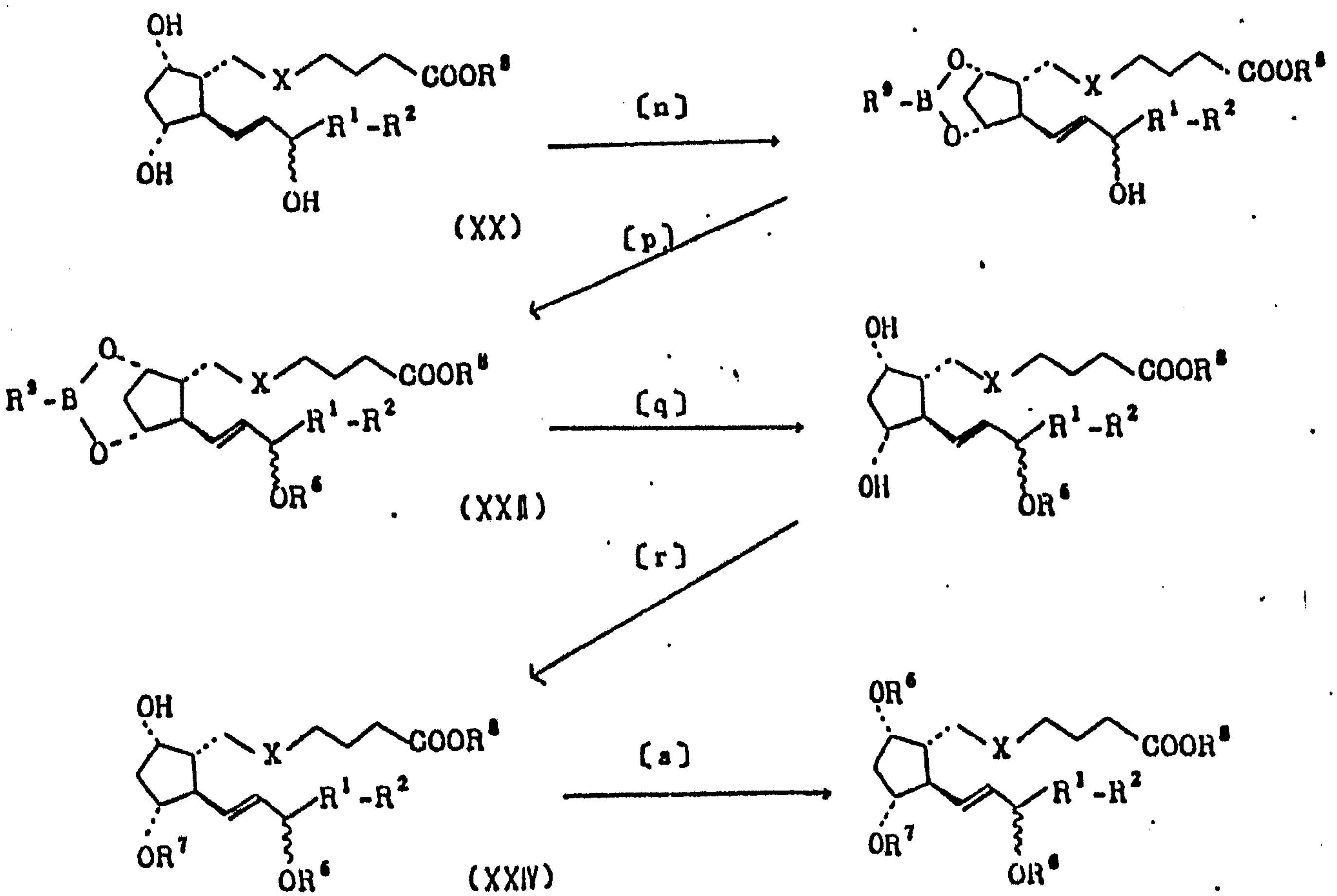


(XX) (XXI)

(XXII) (XXIII)

(XXIV) (VII)

The respective steps in the scheme B are now described. The step [n] can be conducted by reacting with a boric acid corresponding to $R^9$, for example, phenylboric acid [$PhB(OH)_2$] or butylboric acid [$n-BuB(OH)_2$], in an inert organic solvent such as methylene chloride, chloroform, carbon tetrachloride or tetrahydrofuran in the presence of molecular sieves 3A or 4A at reflux temperature [see J.C.S. Chem. Comm., page 658 (1975) and Prostaglandins, Vol. 9, page 109 (1975)].

The step [p] can be conducted using,e.g. 2,3-dihydropyran, 2,3-dihydrofuran, ethyl vinyl ether etc. in an inert organic solvent, for example, methylene chloride, chloroform or diethyl ether, in the presence of a condensing agent, for example, p-toluenesulfonic acid, sulfuric acid or trifluoroacetic acid at a temperature of from room temperature to -30°C. Suitably, it can be conducted using 2,3-dihydropyran in methylene chloride in the presence of pyridine p-toluenesulfonate or p-toluenesulfonic acid at room temperature.

The step [q] can be conducted by reacting with an aqueous hydrogen peroxide solution in an aqueous solution of an alkanol of 1-4 carbon atoms, for example aqueous methanol or aqueous ethanol, at a temperature of not higher than 60°C, preferably at 45-50°C.

The products of steps [n], [p] and [q] can be

used, without purification, in subsequent steps.

The step [r] can be conducted by selectively acylating the hydroxyl group attached to the 11-position of the compound of the general formula (XXIII). Such acylation can be conducted using an appropriate acyl chloride or acid anhydride, for example, benzoyl chloride, in an inert organic solvent, for example, methylene chloride, or in the absence of a solvent, in the presence of a tertiary amine such as pyridine or triethylamine at a temperature of not higher than room temperature, preferably at -30 to -40°C.

The step [s] can be conducted as described for step [p].

According to the present invention, the compounds of general formula (Ib), wherein the various symbols are as hereinbefore defined, may also be prepared by the sequence of reaction steps illustrated by the following scheme C. In the scheme, $R^{10}$ represents an alkylsulfonyl group, preferably containing from 1 to 4 carbon atoms in the alkyl group, or a substituted or unsubstituted arylsulfonyl group, preferably a mesyl group or a tosyl group, and the other symbols are as defined above.

According to a feature of the invention prostaglandin D analogues of general formula (Ib) wherein $R^b$ represents a group of the formula $-COCH_2OH$ and the other symbols are as hereinbefore defined are prepared by the hydrolysis under acidic conditions of a compound of general formula (XXX) depicted in scheme C, wherein the various symbols are as hereinbefore defined.

Scheme C

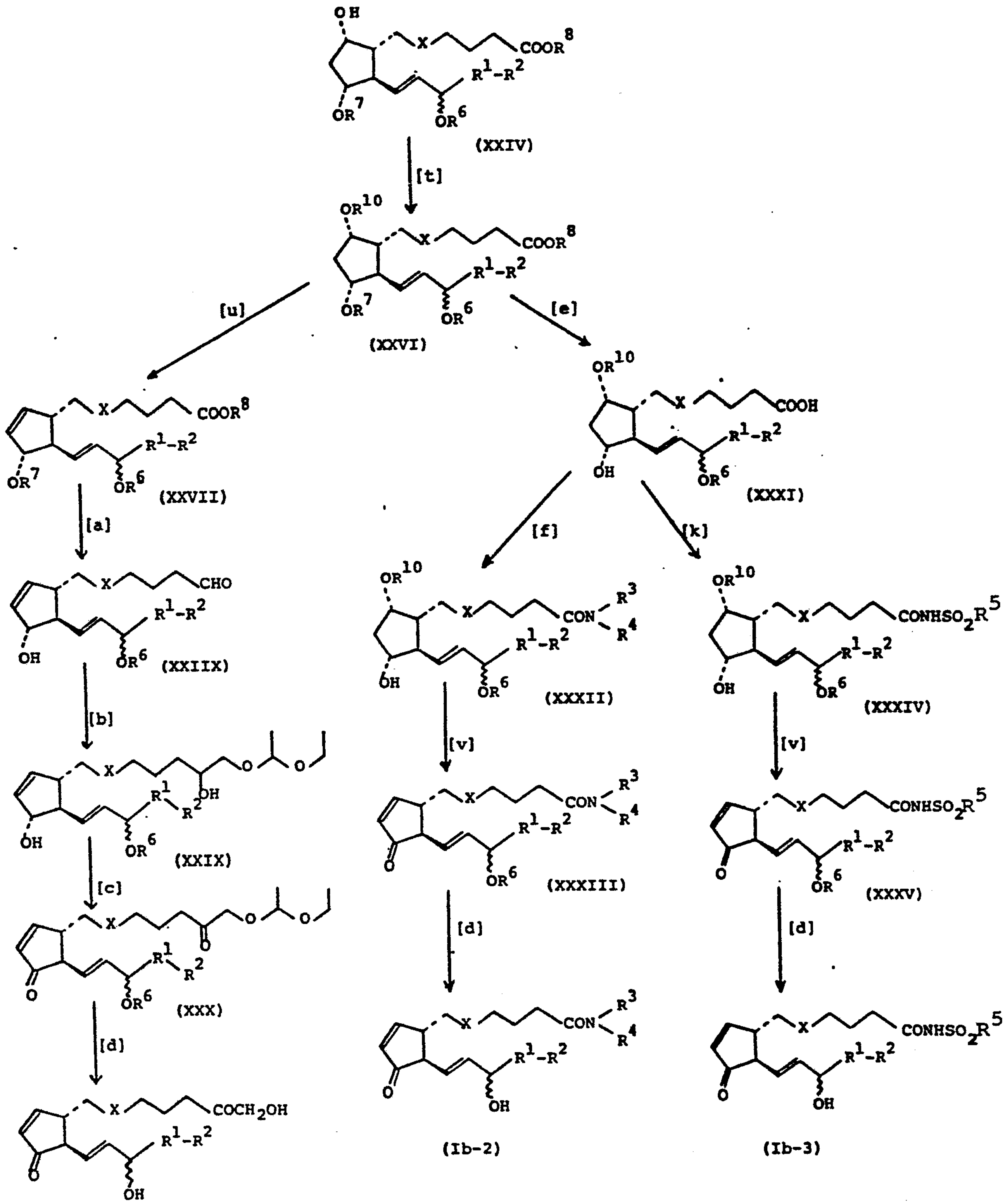


(XXIV)

(XXVI)

(XXVII)

(XXXI)

(XXIIX)

(XXXII)

(XXXIV)

(XXIX)

(XXXIII)

(XXXV)

(XXX)

(Ib-2)

(Ib-3)

(Ib-1)

The respective steps of the scheme C are now described. The steps [a], [b], [c], [d], [e], [f] and [k] may be carried out as hereinbefore described for steps [a], [b], [c], [d], [e], [f] and [k], respectively, in scheme A.

The step [t] may be conducted by reacting with an alkylsulfonyl chloride such as mesyl chloride or an arylsulfonyl chloride such as tosyl chloride at a temperature of from -30°C to 50°C (i) in an inert organic solvent such as methylene chloride in the presence of a tertiary amine such as pyridine or triethylamine, or (ii) in pyridine.

The step [u] may be conducted by reacting with 1,5-diazabicyclo[5.4.0]undec-5-ene (DBU) at a temperature of from 50°C to 100°C.

The step [v] is an oxidation reaction to convert the hydroxyl group into an oxo group, and in addition to the conversion, to eliminate the group $OR^{10}$ and to form a double bond between $C_9$-$C_{10}$ at the same time. The reaction can be conducted as hereinbefore described for step [c].

Thus, according to a further feature of the present invention there is provided a process for the preparation

of prostaglandin D analogues of general formula (Ib), (wherein $R^b$ represents a group of the formula

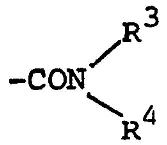 or $-CONHSO_2R^5$ wherein $R^3$, $R^4$ and $R^5$ are as hereinbefore defined and the other symbols are as hereinbefore defined) which comprises the hydrolysis under acidic conditions of a compound of the general formula:

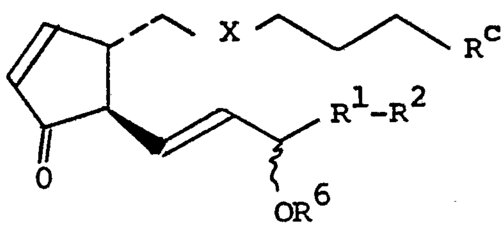

(XXXVI)

wherein $R^c$ represents a group of the formula:

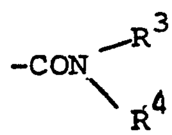 or $-CONHSO_2R^5$

(wherein $R^3$, $R^4$ and $R^5$ and as hereinbefore defined) and the other symbols are as hereinbefore defined. The hydrolysis may be carried out as hereinbefore described for step (d) in scheme A.

According to a further feature of the present invention there is provided a process for the preparation of prostaglandin D analogues of general formula (Ic), wherein the various symbols are as hereinbefore defined, which comprises reacting with an aqueous acid solution a compound of general formula (Ib), (XI), (XV), (XVII),

(XXX) or (XXXVI), wherein the various symbols are as hereinbefore defined: the reaction may be carried out as hereinbefore described for the preparation of a prostaglandin analogue of general formula (Ic) from a compound of general formula (VII).

In the above-described scheme B, the compound of the general formula (XX) employed as the starting material is known as a prostaglandin F analogue, and may be prepared by converting the $OR^6$ groups attached at the 11-position and the 15-position of a compound of the general formula:

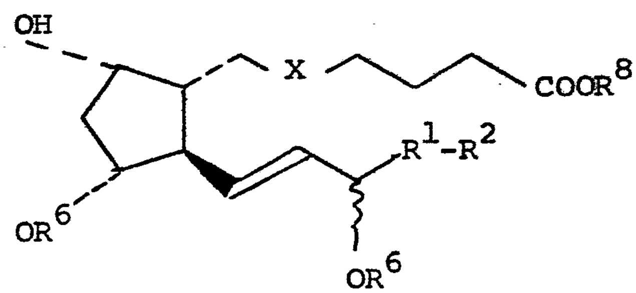

(XXV)

(wherein the various symbols are as hereinbefore defined), to hydroxyl groups under the reaction conditions employed in the above step [d].

The compounds of the general formula (XXV) can be prepared by the application or adaptation of known methods. For example, they can be prepared by:

(1) when the $-R^1-R^2$ group represents a pentyl group, as described in J. Amer. Chem. Soc., 91, 5675 (1969) or ibid. 92, 397 (1970);

(2) when the group $-R^1-R^2$ represents an alkyl group, as described in Japanese Patent Kokai Nos. 42675/72, 54068/73, 64073/73, 124048/74, 95250/75, 96543/75 and 101340/75, British Patent Specification Nos. 1398291, 1483240 and 1540427, United States Patent Specification No. 4024174, and Belgian Patent No. 850084;

(3) when $R^2$ of the group-$R^1-R^2$ represents a cycloalkyl group, as described in Japanese Patent Kokai Nos. 109353/74, 95250/75, 96543/75, 123647/75, 148339/75, 122040/76, 125256/76, 27753/77 and 25544/78, British Patent Specification Nos. 1464916, 1488141, 1483240, 1484210 and 1545213, United States Patent Specifications Nos. 3966792, 4034003, 4024174, 4045468 and 4087620, and Belgian Patent No. 844256;

(4) when $R^2$ of the group $-R^1-R^2$ represents a phenyl group or a phenoxy group, as described in Japanese Patent Kokai Nos. 95250/75, 96543/75, 59841/76, 101961/76 and 25745/77, British Patent Specifications Nos. 1483240 and 1521747, United States Patent Specification Nos. 4024174 and 4065632, and Belgian Patent No. 845348.

The cyclodextrin clathrates of the prostaglandin D analogues of general formula (I) can be prepared using α-, β- or γ-cyclodextrin or a mixture thereof, using the procedure described in the specification of British Patent No. 1351238. Conversion into the cyclodextrin clathrate serves to increase the stability of the prostaglandin D analogue of the general formula (I).

The prostaglandin D analogues of the general formula (I) and their cyclodextrin clathrates either do not show the pharmacological effects typical of other prostaglandins, for example, hypotensive activity, inhibition of platelet aggregation, stimulation of contraction of uterine muscles and the production of diarrhoea or these effects are very weak. In contrast, the anti-tumor effect of the prostaglandin D analogues of general formula (I) is extremely strong and moreover their toxicity is extremely low. They may therefore be employed as very effective anti-tumor agents in the prevention or therapy of leukemia and solid cancer, and in treatment to produce remission thereof.

In an in vitro test on the inhibition of proliferation of human myelogenous leukemic cells (HL-60) or cells originated from human mouth tumor (KB-cells), and in an in vivo test on the prolongation of survival of mice inoculated with Ehrlich ascites tumor, the compounds of the present invention showed an excellent anti-tumor effect. The experimental methods and the results are described below.

Test on Inhibition of Proliferation Using Human Myelogenous Leukemic Cells (HL-60)

(Experimental Method)

The test on the inhibition of proliferation of human myelogenous leukemic cells was conducted by a well known method. The human myelogenous leukemic cells (HL-60) were

added to an RPMI culture solution containing 10% bovine fetal serum, the number of cells in the culture solution was adjusted to $1x10^5$ cells/ml, a solution in ethanol of the test compound was added to give a concentration of 5µg/ml or 1 µg/ml, and the mixture was subjected to stationary culture at 37°C for 4 days. As a control, a culture solution containing 0.1% of ethanol was similarly cultured. The culture solutions were stained by the Trypan Blue staining method, and the surviving cell number was measured to determine the degree of inhibition relative to the control. The results are given in the Table 1.

Test on Inhibition of Proliferation Using Cells Originated from Human Mouth Tumor (KB-cells)

(Experimental Method)

The test on the inhibition of proliferation of cells originated from human mouth tumor was conducted by a well known method. The cells originated from human mouth tumor (KB-cells) were added to Eagle's MEM culture solution

containing 10% bovine fetal serum, the number of cells in the culture solution was adjusted to $1x10^5$ cells/ml, a solution in ethanol of the test compound was added to give a concentration of 5μg/ml or 1μg/ml and the mixture was subjected to stationary culture at 37°C for 4 days. As a control, a culture solution containing 0.1% of ethanol was similarly cultured. After the culture, the culture solutions were stained by the Trypan Blue staining method, and the surviving cell number was measured to determine the degree of inhibition relative to the control.

The results are given in the Table 1.

Table 1

Inhibition of proliferation Using Human Myelogenous Leukemic Cells (HL-60) and Cells Originated from Human Mouth Tumor (KB-cells)

| Test compound | Inhibition (%) | | | |
|---|---|---|---|---|
| | HL-60 cells | | KB-cells | |
| | 5 μg/ml | 1 μg/ml | 5 μg/ml | 1 μg/ml |
| Compound 1 | 96.7 | 20.4 | 100 | 23.5 |
| Compound 2 | 100 | 100 | 100 | 90.9 |
| Compound 3 | 100 | 100 | 100 | 10.6 |
| Compound 4 | 1.5 | 3.9 | 25.5 | 12.6 |
| Compound 5 | 78.1 | 0 | 34.6 | 0.9 |
| Compound 6 | 99.1 | 9.9 | 100 | 19.6 |
| Compound 7 | 100 | 41.8 | 100 | 23.7 |
| Compound 8 | 100 | 30.9 | 100 | 9.5 |
| Compound 9 | 97.8 | 21.5 | 98.8 | 2.8 |
| Compound 10 | | | 100 | 15.4 |
| Compound 11 | | | 100 | 0.9 |

Compound 1 : 2-Decarboxy-2-glycoloyl-$PGD_2$ (prepared in Example 1)

Compound 2 : 2-Decarboxy-2-glycoloyl-9-deoxy-$\Delta^9$-$PGD_2$ (prepared in Example 2)

Compound 3 : (5Z,9Z,14EZ)-1-Hydroxymethylprosta-5,9,12,14-tetraene-1,11-dione (prepared in Example 3)

Compound 4 : 9-Deoxy-$\Delta^9$-$PGD_2$ methanesulfonylamide (prepared in Example 4)

Compound 5 : (5Z,9Z,14EZ)-N-Methanesulfonyl-11-oxoprosta-5,9,12,14-tetraen-1-amide (prepared in Example 5)

Compound 6 : $PGD_2$ N,N-dimethylamide (prepared in Example 6)

Compound 7 : 9-Deoxy-$\Delta^9$-$PGD_2$ N,N-dimethylamide (prepared in Example 7)

Compound 8 : (5Z,9Z,14EZ)-N,N-Dimethyl-11-oxoprosta-5,9,12,14-tetraen-1-amide (prepared in Example 8)

Compound 9 : $PGD_2$ amide (prepared in Example 9)

Compound 10 : 9-Deoxy-$\Delta^9$-$PGD_2$ amide (prerared in Example 10)

Compound 11 : (5Z,9Z,14EZ)-11-Oxoprosta-5,9,12,14-tetraen-1-amide (prepared in Example 11)

Test on Prolongation of Survival of Mice Inoculated with Ehrlich Ascites Tumor

(Experimental Method)

The test on the prolongation of survival of mice inoculated with Ehrlich ascites tumor was conducted by a well known method. ICR mice, weighing 25 - 30 g, were inoculated with Ehrlich ascites tumor cells ($3 \times 10^6$/mouse) intraperitoneally on day 0. A test compound was administered to mice intraperitoneally at a dose of 20 mg/kg/day from day 1 to day 5. Throughout the experiments, each group consisted of six mice (eight mice in control group). The effects of the test compounds were judged by the prolongation of survival time in days. The results are given in the following Table 2. In the Table 2, T/C was calculated below.

$$T/C = \frac{\text{mean survival time in days of treated mice}}{\text{mean survival time in days of control mice}} \times 100$$

Table 2

Prolongation of Survival Time of Mice Inoculated with Ehrlich Ascites Tumor

| Test compound | T/C |
|---|---|
| Compound 1 | 75.3 |
| Compound 2 | 70.5 |
| Compound 3 | 244.6 |
| Compound 11 | 155.6 |

Compounds 1, 2, 3 and 11 are the same as the compounds, 1, 2, 3 and 11 respectively described in Table 1.

The acute toxicity of the compounds of the present invention was more than 100 mg/kg by intraperitoneal administration in mice, so that the PGD analogues of the present invention may be considered to be sufficiently safe and suitable for medical use. For example, $LD_{50}$ values of 2-decarboxy-2-glycoloyl-$PGD_2$ and 9-deoxy-$\Delta^9$-$PGD_2$ amide were 156 mg/kg and 167 mg/kg, respectively, by intraperitoneal administration in mice.

In the compounds represented by the general formula (Ia), preferred examples are as follows: 2-decarboxy-2-glycoloyl-$PGD_2$, 2-decarboxy-2-glycoloyl-16-methyl-$PGD_2$, 2-decarboxy-2-glycoloyl-16,16-dimethyl-$PGD_2$, 2-decarboxy-2-glycoloyl-17,20-dimethyl-$PGD_2$, 2-decarboxy-2-glycoloyl-15-(1-butylcyclobutyl)-16,17,18,19,20-pentanor-$PGD_2$, 2-decarboxy-2-glycoloyl-15-cyclopentyl-16,17,18,19,20-pentanor-$PGD_2$, 2-decarboxy-2-glycoloyl-15-(3-propylcyclopentyl)-16,17,18,19,20-pentanor-$PGD_2$, 2-decarboxy-2-glycoloyl-15-(4-butylcyclohexyl)-16,17,18,19,20-pentanor-$PGD_2$, 2-decarboxy-2-glycoloyl-16-phenyl-17,18,19,20-tetranor-$PGD_2$, 2-decarboxy-2-glycoloyl-16-phenoxy-17,18,19,20-tetranor-$PGD_2$, 2-decarboxy-2-glycoloyl-16-(3-chlorophenoxy)-17,18,19,20-tetranor-$PGD_2$, 2-decarboxy-2-glycoloyl-16-(3-trifluoromethylphenoxy)-17,18,19,20-tetranor-$PGD_2$, $PGD_2$ amide, $PGD_2$ N,N-dimethylamide, 16-methyl-$PGD_2$ N,N-dimethylamide, 16,16-dimethyl-$PGD_2$ N,N-dimethylamide, 17,20-dimethyl-$PGD_2$ N,N-dimethylamide, 15-(1-butylcyclobutyl)-16,17,18,19,20-pentanor-$PGD_2$ N,N-dimethylamide, 15-cyclopentyl-16,17,18,19,20-pentanor-$PGD_2$ N,N-dimethylamide, 15-(3-propylcyclopentyl)-16,17,18,19,20-pentanor-$PGD_2$ N,N-dimethylamide, 15-(4-butylcyclohexyl)-16,17,18,19,20-pentanor-$PGD_2$ N,N-dimethylamide, 16-phenyl-17,18,19,20-tetranor-$PGD_2$ N,N-dimethylamide, 16-phenoxy-17,18,19,20-tetranor-$PGD_2$ N,N-dimethylamide, 16-(3-chlorophenoxy)-17,18,19,20-tetranor-$PGD_2$ N,N-dimethylamide, 16-(3-trifluoromethylphenoxy)-17,18,19,20-tetranor-$PGD_2$ N,N-dimethylamide, and the corresponding $PGD_1$ analogues [i.e. the compounds in which X represents an ethylene group in the general formula (Ia)].

In the compounds represented by the general formula (Ib), preferred examples are as follows: 2-decarboxy-2-glycoloyl-9-deoxy-$\Delta^9$-$PGD_2$, 2-decarboxy-2-glycoloyl-16-methyl-9-deoxy-$\Delta^9$-$PGD_2$, 2-decarboxy-2-glycoloyl-16,16-dimethyl-9-deoxy-$\Delta^9$-$PGD_2$, 2-decarboxy-2-glycoloyl-17,20-dimethyl-9-deoxy-$\Delta^9$-$PGD_2$, 2-decarboxy-2-glycoloyl-15-(1-butylcyclobutyl)-16,17,18,19,20-pentanor-9-deoxy-$\Delta^9$-$PGD_2$, 2-decarboxy-2-glycoloyl-15-cyclopentyl-16,17,18,19,20-pentanor-9-deoxy-$\Delta^9$-$PGD_2$, 2-decarboxy-2-glycoloyl-15-(3-propylcyclopentyl)-16,17,18,19,20-pentanor-9-deoxy-$\Delta^9$-$PGD_2$, 2-decarboxy-2-glycoloyl-15-(4-butylcyclohexyl)-16,17,18,19,20-pentanor-9-deoxy-$\Delta^9$-$PGD_2$, 2-decarboxy-2-glycoloyl-16-phenyl-17,18,19,20-tetranor-9-deoxy-$\Delta^9$-$PGD_2$, 2-decarboxy-2-glycoloyl-16-phenoxy-17,18,19,20-tetranor-9-deoxy-$\Delta^9$-$PGD_2$, 2-decarboxy-2-glycoloyl-16-(3-chlorophenoxy)-17,18,19,20-tetranor-9-deoxy-$\Delta^9$-$PGD_2$, 2-decarboxy-2-glycoloyl-16-(3-trifluoromethylphenoxy)-17,18,19,20-tetranor-9-deoxy-$\Delta^9$-$PGD_2$, 9-deoxy-$\Delta^9$-$PGD_2$ amide, 9-deoxy-$\Delta^9$-$PGD_2$ N,N-dimethylamide, 16-methyl-9-deoxy-$\Delta^9$-$PGD_2$ N,N-dimethylamide, 16,16-dimethyl-9-deoxy-$\Delta^9$-$PGD_2$ N,N-dimethylamide, 17,20-dimethyl-9-deoxy-$\Delta^9$-$PGD_2$ N,N-dimethylamide, 15-(1-butylcyclobutyl)-16,17,18,19,20-pentanor-9-deoxy-$\Delta^9$-$PGD_2$ N,N-dimethylamide, 15-cyclopentyl-16,17,18,19,20-pentanor-9-deoxy-$\Delta^9$-$PGD_2$ N,N-dimethylamide, 15-(3-propylcyclopentyl)-16,17,18,19,20-pentanor-9-deoxy-$\Delta^9$-$PGD_2$ N,N-dimethylamide, 15-(4-butylcyclohexyl)-16,17,18,19,20-pentanor-9-deoxy-$\Delta^9$-$PGD_2$ N,N-dimethylamide, 16-phenyl-17,18,19,20-tetranor-9-deoxy-$\Delta^9$-$PGD_2$ N,N-dimethylamide, 16-phenoxy-17,18,19,20-tetranor-9-deoxy-$\Delta^9$-$PGD_2$ N,N-dimethylamide,

16-(3-chlorophenoxy)-17,18,19,20-tetranor-9-deoxy-$\Delta^9$-$PGD_2$ N,N-dimethylamide, 16-(3-trifluoromethylphenoxy)-17,18,19,20-tetranor-9-deoxy-$\Delta^9$-$PGD_2$ N,N-dimethylamide, 9-deoxy-$\Delta^9$-$PGD_2$ methanesulfonylamide, 16-methyl-9-deoxy-$\Delta^9$-$PGD_2$ methanesulfonylamide, 16,16-dimethyl-9-deoxy-$\Delta^9$-$PGD_2$ methanesulfonylamide, 17,20-dimethyl-9-deoxy-$\Delta^9$-$PGD_2$ methanesulfonylamide, 15-(1-butylcyclobutyl)-16,17,18,19,20-pentanor-9-deoxy-$\Delta^9$-$PGD_2$ methanesulfonylamide, 15-cyclopentyl-16,17,18,19,20-pentanor-9-deoxy-$\Delta^9$-$PGD_2$ methanesulfonylamide, 15-(3-propylcyclopentyl)-16,17,18,19,20-pentanor-9-deoxy-$\Delta^9$-$PGD_2$ methanesulfonylamide, 15-(4-butylcyclohexyl)-16,17,18,19,20-pentanor-9-deoxy-$\Delta^9$-$PGD_2$ methanesulfonylamide, 16-phenyl-17,18,19,20-tetranor-9-deoxy-$\Delta^9$-$PGD_2$ methanesulfonylamide, 16-phenoxy-17,18,19,20-tetranor-9-deoxy-$\Delta^9$-$PGD_2$ methanesulfonylamide, 16-(3-chlorophenoxy)-17,18,19,20-tetranor-9-deoxy-$\Delta^9$-$PGD_2$ methanesulfonylamide, 16-(3-trifluoromethylphenoxy)-17,18,19,20-tetranor-9-deoxy-$\Delta^9$-$PGD_2$ methanesulfonylamide, and the corresponding $PGD_1$ analogues [i.e. the compound in which X represents an ethylene group in the general formula (Ic)].

In the compounds represented by the general formula (Ic), preferred examples are as follows:

(5Z,9Z,14EZ)-1-hydroxymethylprosta-5,9,12,14-tetraene-1,11-dione, (5Z,9Z,14EZ)-1-hydroxymethyl-16-methylprosta-5,9,12,14-tetraene-1,11-dione, (5Z,9Z,14EZ)-1-hydroxymethyl-16,16-dimethylprosta-5,9,12,14-tetraene-1,11-dione, (5Z,9Z,14EZ)-1-hydroxymethyl-17,20-dimethylprosta-5,9,12,14-tetraene-1,11-dione, (5Z,9Z,14EZ)-1-hydroxymethyl-15-(1-butylcyclobutyl)-16,17,18,19,20-pentanorprosta-5,9,12,14-tetraene-1,11-dione, (5Z,9Z,14EZ)-1-hydroxymethyl-15-cyclopentyl-16,17,18,19,20-pentanorprosta-5,9,12,14-tetraene-1,11-dione, (5Z,9Z,14EZ)-1-hydroxymethyl-15-(3-propylcyclopentyl)-16,17,18,19,20-pentanorprosta-5,9,12,14-tetraene-1,11-dione, (5Z,9Z,14EZ)-1-hydroxymethyl-15-(4-butylcyclohexyl)-16,17,18,19,20-pentanorprosta-5,9,12,14-tetraene-1,11-dione, (5Z,9Z,14EZ)-1-hydroxymethyl-16-phenyl-17,18,19,20-tetranorprosta-5,9,12,14-tetraene-1,11-dione, (5Z,9Z,14EZ)-1-hydroxymethyl-16-phenoxy-17,18,19,20-tetranorprosta-5,9,12,14-tetraene-1,11-dione, (5Z,9Z,14EZ)-1-hydroxymethyl-16-(3-chlorophenoxy)-17,18,19,20-tetranorprosta-5,9,12,14-tetraene-1,11-dione, (5Z,9Z,14EZ)-1-hydroxymethyl-16-(3-trifluoromethylphenoxy)-17,18,19,20-tetranorprosta-5,9,12,14-tetraene-1,11-dione, (5Z,9Z,14EZ)-11-oxoprosta-5,9,12,14-tetraen-1-amide, (5Z,9Z,14EZ)-N,N-dimethyl-11-oxoprosta-5,9,12,14-tetraen-1-amide, (5Z,9Z,14EZ)-N,N-dimethyl-11-oxo-16-methylprosta-5,9,12,14-tetraen-1-amide, (5Z,9Z,14EZ)-N,N-dimethyl-11-oxo-16,16-dimethylprosta-5,9,12,14-tetraen-1-amide, (5Z,9Z,14EZ)-N,N-dimethyl-11-oxo-17,20-dimethylprosta-5,9,12,14-tetraen-1-amide, (5Z,9Z,14EZ)-N,N-dimethyl-11-oxo-15-(1-butylcyclobutyl)-16,17,18,19,20-pentanorprosta-5,9,12,14-tetraen-1-amide, (5Z,9Z,14EZ)-N,N-dimethyl-11-oxo-15-cyclopentyl-16,17,18,19,20-pentanorprosta-5,9,12,14-tetraen-1-amide, (5Z,9Z,14EZ)-N,N-dimethyl-11-oxo-15-(3-propylcyclopentyl)-16,17,18,19,20-pentanorprosta-5,9,12,14-tetraen-1-amide, (5Z,9Z,14EZ)-N,N-dimethyl-11-oxo-15-(4-butylcyclohexyl)-16,17,18,19,20-pentanor-

prosta-5,9,12,14-tetraen-1-amide, (5Z,9Z,14EZ)-N,N-dimethyl-11-oxo-16-phenyl-17,18,19,20-tetranorprosta-5,9,12,14-tetraen-1-amide, (5Z,9Z,14EZ)-N,N-dimethyl-11-oxo-16-phenoxy-17,18,19,20-tetranorprosta-5,9,12,14-tetraen-1-amide, (5Z,9Z,14EZ)-N,N-dimethyl-11-oxo-16-(3-chlorophenoxy)-17,18,19,20-tetranorprosta-5,9,12,14-tetraen-1-amide, (5Z,9Z,14EZ)-N,N-dimethyl-11-oxo-16-(3-trifluoromethylphenoxy)-17,18,19,20-tetranorprosta-5,9,12,14-tetraen-1-amide, (5Z,9Z,14EZ)-N-methanesulfonyl-11-oxoprosta-5,9,12,14-tetraen-1-amide, (5Z,9Z,14EZ)-N-methanesulfonyl-11-oxo-16-methylprosta-5,9,12,14-tetraen-1-amide, (5Z,9Z,14EZ)-N-methanesulfonyl-11-oxo-16,16-dimethylprosta-5,9,12,14-tetraen-1-amide, (5Z,9Z,14EZ)-N-methanesulfonyl-11-oxo-17,20-dimethylprosta-5,9,12,14-tetraen-1-amide, (5Z,9Z,14EZ)-N-methanesulfonyl-11-oxo-15-(1-butylcyclobutyl)-16,17,18,19,20-pentanorprosta-5,9,12,14-tetraen-1-amide, (5Z,9Z,14EZ)-N-methanesulfonyl-11-oxo-15-cyclopentyl-16,17,18,19,20-pentanor-

prosta-5,9,12,14-tetraen-1-amide, (5Z,9Z,14EZ)-N-methanesulfonyl-11-oxo-15-(3-propylcyclopentyl)-16,17,18,19,20-pentanorprosta-5,9,12,14-tetraen-1-amide, (5Z,9Z,14EZ)-N-methanesulfonyl-11-oxo-15-(4-butylcyclohexyl)-16,17,18,19,20-pentanorprosta-5,9,12,14-tetraen-1-amide, (5Z,9Z,14EZ)-N-methanesulfonyl-11-oxo-16-phenyl-17,18,19,20-tetranorprosta-5,9,12,14-tetraen-1-amide, (5Z,9Z,14EZ)-N-methanesulfonyl-11-oxo-16-phenoxy-17,18,19,20-tetranorprosta-5,9,12,14-tetraen-1-amide, (5Z,9Z,14EZ)-N-methanesulfonyl-11-oxo-16-(3-chlorophenoxy)-17,18,19,20-tetranorprosta-5,9,12,14-tetraen-1-amide, (5Z,9Z,14EZ)-N-methanesulfonyl-11-oxo-16-(3-trifluoromethylphenoxy)-17,18,19,20-tetranorprosta-5,9,12,14-tetraen-1-amide, and the corresponding compounds in which X represents an ethylene group.

The following Reference Examples and Examples illustrate the preparation of compounds of the present invention. In the Reference Examples and Examples, "TLC", "NMR", "IR" and "Mass" represent "Thin layer chromatography", "Nuclear magnetic resonance spectrum", "Infrared absorption spectrum" and "Mass spectrum", respectively. The solvents in parentheses in chromatography show the developing solvents used: ratios are by volume. Except when specified otherwise, infrared spectra were recorded by the liquid film method and nuclear magnetic resonance spectra were recorded in deuterochloroform ($CDCl_3$) solution.

Reference Example 1

(5Z,13E)-(9α,11α,15S)-9,11-Dihydroxy-15-(tetrahydropyran-2-yloxy)prosta-5,13-dienoic acid methyl ester.

Molecular sieve 4A (20-25 grains) was added to a solution of 1g of $PGF_{2\alpha}$ methyl ester and 409 mg of phenylboric acid in 25 ml of dry methylene chloride, and the mixture was refluxed for one hour. The reaction mixture was cooled to room temperature and filtered.

To the obtained filtrate, 0.382 ml of 2,3-dihydropyran and 70.3 mg of pyridine p-toluenesulfonate were added, and the mixture was stirred for one night. To the reaction mixture, 90 ml of ethyl acetate was added, and the resulting mixture was neutralized with a saturated aqueous solution of sodium bicarbonate, washed with a saturated aqueous solution of sodium chloride, dried over anhydrous magnesium sulfate and concentrated under reduced pressure. To a solution of the residue in 10 ml of methanol, 40 ml of water and 4 ml of a 30% aqueous hydrogen peroxide solution were added, and the reaction mixture was heated to 45-48°C. After cooling to room temperature, the reaction mixture was concentrated under reduced pressure, and the residue was extracted with 50 ml of ethyl acetate. The extract was washed with water and a saturated aqueous solution of sodium chloride, dried over anhydrous magnesium sulfate and concentrated under reduced pressure.

The residue was purified by column chromatography on silica gel using a mixed solvent of cyclohexane and ethyl acetate as an eluent to give 913 mg of the title compound having the following physical characteristics:

TLC(benzene:ethyl acetate = 1:2): Rf = 0.41;

NMR: δ = 5.63-5.16(4H, m), 4.71(1H, m), 3.66(3H, s),
4.24-3.35(5H, m), 0.88(3H, t);

IR : ν = 3440, 2940, 1740, 1432, 1017 $cm^{-1}$;

Mass: m/e = 434, 421, 406.

The following compounds were obtained by the same procedure as described in Reference Example 1:

(a) (13E)-(9α,11α,15S)-9,11-Dihydroxy-15-(tetrahydropyran-2-yloxy)prost-13-enoic acid methyl ester

Starting material: $PGF_{1α}$ methyl ester;

TLC(benzene:ethyl acetate = 1:2): Rf = 0.41;

NMR: δ = 5.7-5.3(2H, m), 4.7(1H, m), 3.6(3H, s),
4.2-3.4(5H, m), 0.9(3H, t);

IR : ν = 3450, 2935, 1737 $cm^{-1}$.

(b) (5Z,13E)-(9α,11α,15S,17S)-9,11-Dihydroxy-15-(tetrahydropyran-2-yloxy)-17,20-dimethylprosta-5,13-dienoic acid methyl ester

Starting material: 17S,20-dimethyl-$PGF_{2α}$ methyl ester;

TLC(benzene:ethyl acetate = 1:2): Rf = 0.43;

NMR: δ =5.7-5.2(4H, m), 4.7(1H, m), 3.6(3H, s),
4.2-3.3(5H, m), 1.0-0.8(6H, m);

IR : ν = 3440, 2938, 1737 $cm^{-1}$;

Mass: m/e = 462.

(c) (5Z,13E)-(9α,11α,15S)-9,11-Dihydroxy-15-(tetrahydropyran-2-yloxy)-15-(3-propylcyclopentyl)-16,17,18,19,20-pentanorprosta-5,13-dienoic acid methyl ester

Starting material: 15-(3-propylcyclopentyl)-16,17,18,19,20-pentanor-$PGF_{2\alpha}$ methyl ester;

TLC(benzene:ethyl acetate = 1:2): Rf = 0.45;

NMR: δ = 5.7-5.2(4H, m), 4.7(1H, m), 3.7(3H, s), 4.2-3.3(5H, m), 0.9(3H, t);

IR : ν = 3440, 2935, 1735 $cm^{-1}$;

Mass: m/e = 474.

(d) (5Z,13E)-(9α,11α,15S)-9,11-Dihydroxy-15-(tetrahydropyran-2-yloxy)-16-phenyl-17,18,19,20-tetranorprosta-5,13-dienoic acid methyl ester

Starting material: 16-phenyl-17,18,19,20-tetranor-$PGF_{2\alpha}$ methyl ester;

TLC(benzene:ethyl acetate = 1:2): Rf = 0.40;

NMR: δ = 7.5-7.1(5H, m), 5.7-5.2(4H, m), 4.7(1H, m), 3.6(3H, s), 4.2-3.3(5H, m);

IR : ν = 3350, 2937, 1737 $cm^{-1}$;

Mass: m/e = 454.

(e) (5Z,13E)-(9α,11α,15R)-9,11-Dihydroxy-15-(tetrahydropyran-2-yloxy)-16-(3-chlorophenoxy)-17,18,19,20-tetranorprosta-5,13-dienoic acid methyl ester

Starting material: 16-(3-chlorophenoxy)-17,18,19,20-tetranor-$PGF_{2\alpha}$ methyl ester;

TLC(benzene:ethyl acetate = 1:2): Rf = 0.40;

NMR: δ = 7.3-6.7(4H, m), 5.7-5.2(4H, m), 4.7-3.8(8H, m), 3.7(3H, s);

IR : ν = 3440, 2935, 1737, 1600 $cm^{-1}$;

Mass: m/e = 504.

Reference Example 2

(5Z,13E)-(9α,11α,15S)-9-Hydroxy-11-benzoyloxy-15-(tetrahydropyran-2-yloxy)prosta-5,13-dienoic acid methyl ester

To a solution of 913 mg of 9,11-dihydroxy compound (prepared in Reference Example 1) in 40 ml of methylene chloride, 1.63 ml of pyridine was added. After cooling the mixture to -30 to-40°C, a solution of 0.70 ml of benzoyl chloride in 1.83 ml of methylene chloride was slowly added dropwise and then 485 μl of methanol was added. The reaction mixture was allowed to stand for 20 minutes, warmed to -20°C, 3.1 g of sodium bisulfate monohydrate was added, and the reaction mixture was warmed to room temperature and stirred for 30 minutes. To the reaction mixture, 20 ml of methylene chloride was added, washed with water and a saturated aqueous solution of sodium chloride, dried over anhydrous magnesium sulfate and concentrated under reduced pressure. The residue was purified by column chromatography on silica gel using a mixed solvent of ethyl acetate and cyclohexane as an eluent to give 942 mg of the title compound having the following physical characteristics:

TLC(cyclohexane:ethyl acetate = 2:1): Rf = 0.46;

NMR: δ = 8.1-7.8(2H, m), 7.6-7.2(3H, m), 5.8-5.2(4H, m), 5.2-4.9(1H, m), 4.5(1H, m), 3.6(3H, s), 0.85(3H, t);

IR : ν = 3520, 2940, 1735, 1720, 1277 $cm^{-1}$.

The following compounds were obtained by the same procedure as described in Reference Example 2:

(a) (13E)-(9α,11α,15S)-9-Hydroxy-11-benzoyloxy-15-(tetrahydropyran-2-yloxy)prost-13-enoic acid methyl ester

Starting material: 9,11-dihydroxy compound prepared in Reference Example 1(a);

TLC(cyclohexane:ethyl acetate = 2:1): Rf = 0.46;

NMR: δ = 8.1-7.8(2H, m), 7.6-7.2(3H, m), 5.8-5.2(2H, m), 5.1(1H, m), 4.6(1H, m), 3.6(3H, s), 0.88(3H, t);

IR : ν = 3520, 2935, 1738, 1720, 1275 $cm^{-1}$;

(b) (5Z,13E)-(9α,11α,15S,17S)-9-Hydroxy -11-benzoyloxy-15-(tetrahydropyran-2-yloxy)-17,20-dimethylprosta-5,13-dienoic acid methyl ester

Starting material: 9,11-dihydroxy compound prepared in Reference Example 1(b);

TLC(cyclohexane:ethyl acetate = 2:1): Rf = 0.48;

NMR: δ = 8.1-7.8(2H, m), 7.6-7.2(3H, m), 5.8-5.2(4H, m), 5.2-4.9(1H, m), 4.5(1H, m), 3.6(3H, s), 1.0-0.8(6H, m);

IR : ν = 3530, 2940, 1737, 1720, 1275 $cm^{-1}$.

(c) (5Z,13E)-(9α,11α,15S)-9-Hydroxy-11-benzoyloxy-15-(tetrahydropyran-2-yloxy)-15-(3-propylcyclopentyl)-16,17,18,19,20-pentanorprosta-5,13-dienoic acid methyl ester

Starting material: 9,11-dihydroxy compound prepared in Reference Example 1(c);

TLC(cyclohexane:ethyl acetate = 2:1): Rf = 0.50;

NMR: δ = 8.1-7.8(2H, m), 7.6-7.2(3H, m), 5.8-5.2(4H, m), 5.2-4.9(1H, m), 4.6(1H, m), 3.6(3H, s), 0.9(3H, t);

IR : ν =3530, 2935, 1735, 1720 $cm^{-1}$.

(d) (5Z,13E)-(9α,11α,15S)-9-Hydroxy-11-benzoyloxy-15-(tetrahydropyran-2-yloxy)-16-phenyl-17,18,19,20-tetranorprosta-5,13-dienoic acid methyl ester

Starting material: 9,11-dihydroxy compound prepared in Reference Example 1(d);

TLC(cyclohexane:ethyl acetate = 2:1): Rf = 0.45;

NMR: δ = 8.1-7.1(10H, m), 5.8-5.2(4H, m), 5.2-4.9(1H, m), 4.6(1H, m), 3.6(3H, s);

IR : ν = 3540, 2935, 1737, 1720 $cm^{-1}$.

(e) (5Z,13E)-(9α,11α,15R)-9-Hydroxy-11-benzoyloxy-15-(tetrahydropyran-2-yloxy)-16-(3-chlorophenoxy)-17,18,19,20-tetranorprosta-5,13-dienoic acid methyl ester

Starting material: 9,11-dihydroxy compound prepared in Reference Example 1(e);

TLC(cyclohexane:ethyl acetate = 2:1): Rf = 0.45;

NMR: δ = 8.1-7.8(2H, m), 7.6-6.7(7H, m), 5.8-5.2(4H, m), 5.2-4.9(1H, m), 3.7(3H, s);

IR : ν = 3530, 2935, 1736, 1720, 1600, 1273 $cm^{-1}$.

Reference Example 3

(5Z,13E)-(9α,11α,15S)-9,15-Bis(tetrahydropyran-2-yloxy)-11-benzoyloxyprosta-5,13-dienoic acid methyl ester

Under an atmosphere of nitrogen, 1 ml of 2,3-dihydropyran was added dropwise to a solution of 3.77 g of the 9-hydroxy compound (prepared in Reference Example 2) in 15 ml of methylene chloride at room temperature and then a trace amount of p-toluenesulfonic acid was added, and the resulting mixture was stirred for 15 minutes, 0.1 ml of triethylamine was added, and concentrated under reduced pressure. The residue was purified by column chromatography on silica gel using a mixed solvent of n-hexane and ethyl acetate as an eluent to give 3.95 g of the title compound having the following physical characteristics:

TLC(n-hexane:ethyl acetate = 2:1): Rf = 0.45;

NMR: δ = 8.1-7.9(2H, m), 7.6-7.3(3H, m), 5.7-5.3(4H, m), 5.3-5.0(1H, m), 4.7-4.4(2H, m), 3.7(3H, s), 0.8(3H, t);

IR : ν = 2940, 1735, 1720, 1450, 1270, 1110, 1020 $cm^{-1}$.

The following compounds were obtained by the same procedure as described in Reference Example 3:

(a) (13E)-(9α,11α,15S)-9,15-Bis(tetrahydropyran-2-yloxy)-11-benzoyloxyprost-13-enoic acid methyl ester

Starting material: 9-hydroxy compound prepared in Reference Example 2(a);

TLC(n-hexane:ethyl acetate = 2:1): Rf = 0.46;

NMR: δ = 8.1-7.8(2H, m), 7.5-7.2(3H, m), 5.6-4.7(3H, m), 4.7-4.3(2H, m), 3.6(3H, s), 0.9(3H, t);

IR : ν = 2940, 1740, 1720, 1277 $cm^{-1}$.

(b) (5Z,13E)-(9α,11α,15S,17S)-9,15-Bis(tetrahydropyran-2-yloxy)-11-benzoyloxy-17,20-dimethylprosta-5,13-dienoic acid methyl ester

Starting material: 9-hydroxy compound prepared in Reference Example 2(b);

TLC(n-hexane:ethyl acetate = 2:1): Rf = 0.47;

NMR: δ = 8.1-7.9(2H, m), 7.6-7.2(3H, m), 5.7-5.2(4H, m), 5.2-4.9(1H, m), 4.75-4.4(2H, m), 3.7(3H, s), 1.0-0.7(6H, m);

IR : ν = 2940, 1736, 1720 $cm^{-1}$.

(c) (5Z,13E)-(9α,11α,15S)-9,15-Bis(tetrahydropyran-2-yloxy)-11-benzoyloxy-15-(3-propylcyclopentyl)-16,17,18,19,20-pentanorprosta-5,13-dienoic acid methyl ester

Starting material: 9-hydroxy compound prepared in Reference Example 2(c);

TLC(n-hexane:ethyl acetate = 2:1): Rf = 0.49;

NMR: δ = 8.1-7.9(2H, m), 7.6-7.2(3H, m), 5.7-5.2(4H, m), 5.3-4.9(1H, m), 4.8-4.4(2H, m), 3.7(3H, s), 0.89(3H, t);

IR : ν = 2940, 1735, 1722 $cm^{-1}$.

(d) (5Z,13E)-(9α,11α,15S)-9,15-Bis(tetrahydropyran-2-yloxy)-11-benzoyloxy-16-phenyl-17,18,19,20-tetranorprosta-5,13-dienoic acid methyl ester

Starting material: 9-hydroxy compound prepared in Reference Example 2(d);

TLC(n-hexane:ethyl acetate = 2:1): Rf = 0.45;

NMR: δ = 8.1-7.1(10H, m), 5.7-5.2(4H, m), 5.2-4.9(1H, m), 4.7-4.5(2H, m), 3.7(3H, s);

IR : ν = 2935, 1736, 1720 $cm^{-1}$.

(e) (5Z,13E)-(9α,11α,15R)-9,15-Bis(tetrahydropyran-2-yloxy)-11-benzoyloxy-16-(3-chlorophenoxy)-17,18,19,20-tetranorprosta-5,13-dienoic acid methyl ester

Starting material: 9-hydroxy compound prepared in Reference Example 2(e);

TLC(n-hexane:ethyl acetate = 2:1): Rf = 0.45;

NMR: δ = 8.1-7.9(2H, m), 7.6-6.7(7H, m), 5.7-5.2(4H, m), 5.2-4.9(1H, m), 3.7(3H, s);

IR : ν = 2940, 1737, 1720, 1600 $cm^{-1}$.

Reference Example 4

(5Z,13E)-(9α,11α,15S)-9,15-Bis(tetrahydropyran-2-yloxy)-11-hydroxyprosta-5,13-dien-1-al

Under an atomosphere of nitrogen, 1.3 ml of a 25% (w/v) solution of diisobutylaluminum hydride in toluene was added dropwise to a solution of 500 mg of ester compound (prepared in Reference Example 3) in 25 ml of toluene at -78°C, and the mixture was stirred for one hour. To the reaction mixture, 0.5 ml of methanol was added dropwise, and the resulting mixture was warmed to 0°C. To the reaction mixture, 1.3 ml of water was added, and the mixture was warmed to room temperature with vigorous stirring and stirred for one hour. The reaction mixture was filtered, and the filtrate was concentrated under reduced pressure. The residue was purified by column chromatography on silica gel using a mixed solvent of n-hexane and ethyl acetate as an eluent to give 311 mg of the title compound having the following physical characteristics:

TLC(n-hexane:ethyl acetate = 1:1): Rf = 0.21;

NMR: δ = 9.79(1H,t), 5.7-5.1(4H, m), 4.8-4.4(2H, m),
1.0-0.7(3H, m);

IR : ν = 3450, 2930, 2710, 1735, 1730, 1450, 1240, 1020 $cm^{-1}$;

Mass: m/e = 404, 302.

The following compounds were obtained by the same procedure as described in Reference Example 4:

(a) (13E)-(9α,11α,15S)-9,15-Bis(tetrahydropyran-2-yloxy)-11-hydroxyprost-13-en-1-al

Starting material: ester compound prepared in Reference Example 3(a)

TLC(n-hexane:ethyl acetate = 1:1): Rf = 0.22;

NMR: δ = 9.9(1H, t), 5.6-5.2(2H, m), 4.8-4.5(2H, m),
4.3-3.2(7H, m), 0.88(3H, t);

IR : ν = 3460, 2940, 1740, 1020 $cm^{-1}$;

Mass: m/e = 406, 304.

(b) (5Z,13E)-(9α,11α,15S,17S)-9,15-Bis(tetrahydropyran-2-yloxy)-11-hydroxy-17,20-dimethylprosta-5,13-dien-1-al

Starting material: ester compound prepared in Reference Example 3(b);

TLC(n-hexane:ethyl acetate = 1:1): Rf = 0.23;

NMR: δ = 7.8(1H, t), 5.7-5.1(4H, m), 4.8-4.4(2H, m), 1.0-0.7(6H, m);

IR : ν = 3450, 2930, 2710, 1735, 1728 $cm^{-1}$;

Mass: m/e = 432, 330.

(c) (5Z,13E)-(9α,11α,15S)-9,15-Bis(tetrahydropyran-2-yloxy)-11-hydroxy-15-(3-propylcyclopentyl)-16,17,18,19,20-pentanorprosta-5,13-dien-1-al

Starting material: ester compound prepared in Reference Example 3(c);

TLC(n-hexane:ethyl acetate = 1:1): Rf = 0.23;

NMR: δ = 9.76(1H, t), 5.7-5.2(4H, m), 4.8-4.4(2H, m), 1.0-0.7(3H, t);

IR : ν = 3450, 2928, 1736 $cm^{-1}$;

Mass: m/e = 444.

(d) (5Z,13E)-(9α,11α,15S)-9,15-Bis(tetrahydropyran-2-yloxy)-11-hydroxy-16-phenyl-17,18,19,20-tetranorprosta-5,13-dien-1-al

Starting material: ester compound prepared in Reference Example 3(d);

TLC(n-hexane:ethyl acetate = 1:1): Rf = 0.20;

NMR: δ = 9.81(1H, t), 7.5-7.1(5H, m), 4.7-4.4(2H, m);

IR : ν = 3450, 2930, 1734 $cm^{-1}$;

Mass: m/e = 424, 322.

(e) (5Z,13E)-(9α,11α,15R)-9,15-Bis(tetrahydropyran-2-yloxy)-11-hydroxy-16-(3-chlorophenoxy)-17,18,19,20-tetranor-prosta-5,13-dien-1-al

Starting material: ester compound prepared in Reference Example 3(e);

TLC(n-hexane:ethyl acetate = 1:1): Rf = 0.19;

NMR: δ = 9.82(1H, t), 7.3-6.7(4H, m), 4.8-3.7(11H, m);

IR : ν = 3440, 2930, 1735, 1730, 1600 $cm^{-1}$;

Mass: m/e = 474, 372.

Reference Example 5

(5Z,13E)-(1RS,9α,11α,15S)-1-(2,4-Dioxa-3-methylhexyl)-9,15-bis(tetrahydropyran-2-yloxy)prosta-5,13-diene-1,11-diol

Under an atomosphere of nitrogen, 1.2 ml of a 1.6 M solution of n-butyl lithium in n-hexane was added dropwise to a solution of 707 mg of (2,4-dioxa-3-methylhexyl)-tri-n-butylstannane [prepared by the same procedure as described in J. Am. Chem. Soc., 100, 1481 (1978)] in 20 ml of tetrahydrofuran at -78°C, and the resulting mixture was stirred for 15 minutes. To the solution obtained, 311 mg of 1-aldehyde compound (prepared in Reference Example 4) in 3 ml of tetrahydrofuran was slowly added dropwise, and the resulting mixture was stirred for 15 minutes and warmed to 0°C. The reaction mixture was poured into an aqueous solution of ammonium chloride and extracted with ethyl acetate. The extract was washed with a saturated aqueous solution of sodium chloride, dried, and concentrated under reduced pressure.

The residue was purified by column chromatography on silica gel using a mixed solvent of n-hexane and ethyl acetate firstly and ethyl acetate finally as an eluent to give 277 mg of the title compound having the following physical characteristics:

TLC(n-hexane:ethyl acetate = 1:3): Rf = 0.21;

NMR: δ = 5.7-5.3(4H, m), 4.9-4.5(3H, m), 1.05-0.8(3H, m);

IR : ν = 3450, 2940, 1130, 1020 $cm^{-1}$;

Mass: m/e = 508.

The following compounds were obtained by the same procedure as described in Reference Example 5:

(a) (13E)-(1RS,9α,11α,15S)-1-(2,4-Dioxa-3-methylhexyl)-9,15-bis(tetrahydropyran-2-yloxy)prost-13-ene-1,11-diol

Starting material: 1-aldehyde compound prepared in Reference Example 4(a);

TLC(n-hexane:ethyl acetate = 1:3): Rf = 0.23;

NMR: δ = 5.6-5.3(2H, m), 4.8-4.5(3H, m), 4.4-3.3(11H, m), 0.9(3H, t);

IR : ν = 3440, 2940, 1132, 1020 $cm^{-1}$;

Mass: m/e = 510.

(b) (5Z,13E)-(1RS,9α,11α,15S,17S)-1-(2,4-Dioxa-3-methylhexyl)-9,15-bis(tetrahydropyran-2-yloxy)-17,20-dimethylprosta-5,13-diene-1,11-diol

Starting material: 1-aldehyde compound prepared in Reference Example 4(b);

TLC(n-hexane:ethyl acetate = 1:3): Rf = 0.23;

NMR: δ = 5.7-5.3(4H, m), 4.9-4.5(3H, m), 1.1-0.8(6H, m);

IR : ν = 3450, 2938, 1133 $cm^{-1}$;

Mass: m/e = 536.

(c) (5Z,13E)-(1RS,9α,11α,15S)-1-(2,4-Dioxa-3-methylhexyl)-9,15-bis(tetrahydropyran-2-yloxy)-15-(3-propylcyclopentyl)-16,17,18,19,20-pentanorprosta-5,13-diene-1,11-diol

Starting material: 1-aldehyde compound prepared in Reference Example 4(c);

TLC(n-hexane:ethyl acetate = 1:3): Rf = 0.22;

NMR: δ = 5.7-5.3(4H, m), 4.8-4.5(3H, m), 1.05-0.8(3H, m);

IR : ν = 3445, 2937, 1132, 1022 $cm^{-1}$;

Mass: m/e = 548.

(d) (5Z,13E)-(1RS,9α,11α,15S)-1-(2,4-Dioxa-3-methylhexyl)-9,15-bis(tetrahydropyran-2-yloxy)-16-phenyl-17,18,19,20-tetranorprosta-5,13-diene-1,11-diol

Starting material: 1-aldehyde compound prepared in Reference Example 4(d);

TLC(n-hexane:ethyl acetate = 1:3): Rf = 0.19;

NMR: δ = 7.5-7.1(5H, m), 5.7-5.3(4H, m), 4.8-4.5(3H, m);

IR : ν = 3440, 2938 $cm^{-1}$;

Mass: m/e = 528.

(e) (5Z,13E)-(1RS,9α,11α,15R)-1-(2,4-Dioxa-3-methylhexyl)-9,15-bis(tetrahydropyran-2-yloxy)-16-(3-chlorophenoxy)-17,18,19,20-tetranorprosta-5,13-diene-1,11-diol

Starting material: 1-aldehyde compound prepared in Reference Example 4(e);

TLC(n-hexane:ethyl acetate = 1:3): Rf = 0.19;

NMR: δ = 7.3-6.7(4H, m), 5.7-5.3(4H, m);

IR : ν = 3450, 2937, 1600 $cm^{-1}$;

Mass: m/e = 578.

Reference Example 6

(5Z,13E)-(9α,15S)-1-(2,4-Dioxa-3-methylhexyl)-9,15-bis(tetrahydropyran-2-yloxy)prosta-5,13-diene-1,11-dione

Under an atomosphere of nitrogen, 1.1 ml of pyridine and 660 mg of chromium trioxide were added to 20 ml of methylene chloride at room temperature, and the resulting mixture was stirred for 15 minutes. To the reaction mixture, 3 g of Celite was added at the same temperature and 200 mg of 1,11-diol compound (prepared in Reference Example 5) in 2 ml of methylene chloride was then added at 0°C. The mixture obtained was warmed to room temperature and stirred for 30 minutes. The reaction mixture was diluted with ethyl acetate and filtered. The obtained filtrate was washed with water, a 5% aqueous solution of cupric sulfate and a saturated aqueous solution of sodium chloride, dried, and concentrated under reduced pressure to give the crude title compound having the following physical characteristics. The product obtained was used in the next reaction without purification.

TLC(n-hexane:ethyl acetate = 1:1): Rf = 0.36;

IR : $\nu$ = 2930, 1740, 1720, 1125, 1075, 1020, 980 $cm^{-1}$.

The following compounds were obtained by the same procedure as described in Reference Example 6:

(a) (13E)-(9α,15S)-1-(2,4-Dioxa-3-methylhexyl)-9,15-bis(tetrahydropyran-2-yloxy)prost-13-ene-1,11-dione

Starting material: 1,11-diol compound prepared in Reference Example 5(a);

TLC(n-hexane:ethyl acetate = 1:1): Rf = 0.37;

IR : $\nu$ = 2940, 1740, 1720, 1130, 1020 $cm^{-1}$.

(b) (5Z,13E)-(9α,15S,17S)-1-(2,4-Dioxa-3-methylhexyl)-9,15-bis(tetrahydropyran-2-yloxy)-17,20-dimethylprosta-5,13-diene-1,11-dione

Starting material: 1,11-diol compound prepared in Reference Example 5(b);

TLC(n-hexane:ethyl acetate = 1:1): Rf = 0.38;

IR : ν = 2930, 1740, 1721 $cm^{-1}$.

(c) (5Z,13E)-(9α,15S)-1-(2,4-Dioxa-3-methylhexyl)-9,15-bis(tetrahydropyran-2-yloxy)-15-(3-propylcyclopentyl)-16,17,18,19,20-pentanorprosta-5,13-diene-1,11-dione

Starting material: 1,11-diol compound prepared in Reference Example 5(c);

TLC(n-hexane:ethyl acetate = 1:1): Rf = 0.38;

IR : ν = 2932, 1740, 1720 $cm^{-1}$.

(d) (5Z,13E)-(9α,15S)-1-(2,4-Dioxa-3-methylhexyl)-9,15-bis(tetrahydropyran-2-yloxy)-16-phenyl-17,18,19,20-tetranorprosta-5,13-diene-1,11-dione

Starting material: 1,11-diol compound prepared in Reference Example 5(d);

TLC(n-hexane:ethyl acetate = 1:1): Rf = 0.34;

IR : ν = 2930, 1740, 1720 $cm^{-1}$.

(e) (5Z,13E)-(9α,15R)-1-(2,4-Dioxa-3-methylhexyl)-9,15-bis(tetrahydropyran-2-yloxy)-16-(3-chlorophenoxy)-17,18,19,20-tetranorprosta-5,13-diene-1,11-dione

Starting material: 1,11-diol compound prepared in Reference Example 5(e);

TLC(n-hexane:ethyl acetate = 1:1): Rf = 0.34;

IR : ν = 2930, 1740, 1720, 1600 $cm^{-1}$.

Example 1

(5Z,13E)-(9α,15S)-1-Hydroxymethyl-9,15-dihydroxyprosta-5,13-diene-1,11-dione (i.e. 2-Decarboxy-2-glycoloyl-$PGD_2$)

To a solution of 200 mg of 1-(2,4-dioxa-3-methylhexyl)-9,15-bis(tetrahydropyran-2-yloxy) compound (prepared in Reference Example 6) in 0.5 ml of tetrahydrofuran, 5 ml of a 65% (v/v) aqueous acetic acid was added, and the resulting mixture was stirred for 10 minutes at 70°C. After cooling with an ice-bath,the reaction mixture was diluted with ice-water and extracted with ethyl acetate. The extract was washed with a saturated aqueous solution of sodium chloride,dried, and concentrated with addition of dry toluene under reduced pressure at low temperature. The residue was purified by column chromatography on silica gel using a mixed solvent of n-hexane and ethyl acetate firstly and ethyl acetate finally as an eluent to give 85 mg of the title compound having the following physical characteristics:

TLC(chloroform:tetrahydrofuran:acetic acid = 10:2:1): Rf = 0.17;

NMR: δ = 5.74-5.35(4H, m), 4.56-4.42(1H, m), 4.24(2H, s), 4.20-4.02(1H, m), 2.94-2.74(1H, dd), 0.88(3H, t);

IR : ν = 3430, 1725, 1400, 1275, 1070, 1020, 970 $cm^{-1}$;

Mass: m/e = 366($M^+$), 348, 330.

The following compounds were obtained by the same procedure as described in Example 1:

(a) (13E)-(9α,15S)-1-Hydroxymethyl-9,15-dihydroxyprost-13-ene-1,11-dione (i.e. 2-Decarboxy-2-glycoloyl-$PGD_1$)

Starting material: 1-(2,4-dioxa-3-methylhexyl)-9,15-bis(tetrahydropyran-2-yloxy) compound prepared in Reference Example 6(a);

TLC(chloroform:tetrahydrofuran:acetic acid = 10:2:1):

Rf = 0.17;

NMR: δ = 5.73-5.35(2H, m), 4.47(1H, m), 4.25(2H, s), 4.10(1H, m), 0.88(3H, t);

IR : ν = 3430, 2930, 1722, 1073 $cm^{-1}$;

Mass: m/e = 350, 332.

(b) (5Z,13E)-(9α,15S,17S)-1-Hydroxymethyl-9,15-dihydroxy-17,20-dimethylprosta-5,13-diene-1,11-dione (i.e. 2-Decarboxy-2-glycoloyl-17S,20-dimethyl-$PGD_2$)

Starting material: 1-(2,4-dioxa-3-methylhexyl)-9,15-bis(tetrahydropyran-2-yloxy) compound prepared in Reference Example 6(b);

TLC(chloroform:tetrahydrofuran:acetic acid = 10:2:1):

Rf = 0.19;

NMR: δ = 5.75-5.34(4H, m), 4.58-4.40(1H, m), 4.23(2H, s), 4.19-4.03(1H, m), 2.95-2.73(1H, dd), 1.0-0.8(6H, m);

IR : ν = 3435, 1728, 1403 $cm^{-1}$;

Mass: m/e = 394($M^+$), 376, 358.

(c) (5Z,13E)-(9α,15S)-1-Hydroxymethyl-9,15-dihydroxy-15-(3-propylcyclopentyl)-16,17,18,19,20-pentanorprosta-5,13-diene-1,11-dione (i.e. 2-Decarboxy-2-glycoloyl-15-(3-propylcyclopentyl)-16,17,18,19,20-pentanor-$PGD_2$)

Starting material: 1-(2,4-dioxa-3-methylhexyl)-9,15-bis(tetrahydropyran-2-yloxy) compound prepared in Reference Example 6(c);

TLC(chloroform:tetrahydrofuran:acetic acid = 10:2:1):

Rf = 0.19;

NMR: δ = 5.74-5.34(4H, m), 4.47(1H, m), 4.26(2H, s), 4.10(1H, m), 1.0-0.8(3H, m);

IR : ν = 3430, 1727 $cm^{-1}$;

Mass: m/e = 388, 370.

(d) (5Z,13E)-(9α,15S)-1-Hydroxymethyl-9,15-dihydroxy-16-phenyl-17,18,19,20-tetranorprosta-5,13-diene-1,11-dione (i.e. 2-Decarboxy-2-glycoloyl-16-phenyl-17,18,19,20-tetranor-$PGD_2$)

Starting material: 1-(2,4-dioxa-3-methylhexyl)-9,15-bis(tetrahydropyran-2-yloxy) compound prepared in Reference Example 6(d);

TLC(chloroform:tetrahydrofuran:acetic acid = 10:2:1): Rf = 0.16;

NMR: δ = 7.5-7.1(5H, m), 5.72-5.34(4H, m), 4.53(1H, m), 4.28(2H, s);

Mass: m/e = 386($M^+$), 368, 350.

(e) (5Z,13E)-(9α,15R)-1-Hydroxymethyl-9,15-dihydroxy-16-(3-chlorophenoxy)-17,18,19,20-tetranorprosta-5,13-diene-1,11-dione [i.e. 2-Decarboxy-2-glycoloyl-16-(3-chlorophenoxy)-17,18,19,20-tetranor-$PGD_2$]

Starting material: 1-(2,4-dioxa-3-methylhexyl)-9,15-bis(tetrahydropyran-2-yloxy) compound prepared in Reference Example 6(e);

TLC(chloroform:tetrahydrofuran:acetic acid = 10:2:1): Rf = 0.16;

NMR: δ = 7.3-6.7(4H, m), 5.76-5.33(4H, m), 4.56(1H, m), 4.27(2H, s);

IR : ν = 3430, 1726, 1600 $cm^{-1}$;

Mass: m/e = 436($M^+$), 418, 400.

Example 2

(5Z,9Z,13E)-(15S)-1-Hydroxymethyl-15-hydroxyprosta-5,9,13-triene-1,11-dione (i.e. 2-Decarboxy-2-glycoloyl-9-deoxy-$\Delta^9$-$PGD_2$)

To a solution of 51 mg of $PGD_2$ compound (prepared in Example 1) in 0.7 ml of ethanol, 70 ml of Tris-hydrochloric acid buffer solution [prepared from 17.5 ml of a 0.2M aqueous solution of Tris(hydroxymethyl)aminomethane, 31.5 ml of 0.1N aqueous hydrochloric acid and 21 ml of water] (pH 7.2) was added, and the resulting solution was stirred for 48 hours at 37°C and extracted with ethyl acetate. The extract was washed with a saturated aqueous solution of sodium chloride, dried, and concentrated under reduced pressure. The residue was purified by column chromatography on silica gel using a mixed solvent of n-hexane and ethyl acetate as an eluent to give 4 mg of the title compound having the following physical characteristics.

TLC(chloroform:tetrahydrofuran:acetic acid = 10:2:1): Rf = 0.57;

NMR: $\delta$ = 7.58(1H, dd), 6.17(1H, dd), 5.7-5.3(4H, m), 4.23(2H, bs), 4.16-4.04(1H, m), 2.92-2.76(1H, m), 2.70-2.56(1H, m), 1.0-0.8(3H, m);

Mass: m/e = 348($M^+$), 330.

The following compounds were obtained by the same procedure as described in Example 2:

(a) (9Z,13E)-(15S)-1-Hydroxymethyl-15-hydroxyprosta-9,13-diene-1,11-dione (i.e. 2-Decarboxy-2-glycoloyl-9-deoxy-$\Delta^9$-$PGD_1$)

Starting material: $PGD_1$ compound prepared in Example 1(a);

TLC(chloroform:tetrahydrofuran:acetic acid = 10:2:1): Rf = 0.60;

NMR: δ = 7.57(1H, dd), 6.17(1H, dd), 5.7-5.4(2H, m), 4.24(2H, s), 4.10(1H, m), 0.88(3H, t);

Mass: m/e = 350($M^+$), 332.

(b) (5Z,9Z,13E)-(15S,17S)-1-Hydroxymethyl-15-hydroxy-17,20-dimethylprosta-5,9,13-triene-1,11-dione (i.e. 2-Decarboxy-2-glycoloyl-17S,20-dimethyl-9-deoxy-$\Delta^9$-$PGD_2$)

Starting material: $PGD_2$ compound prepared in Example 1(b);

TLC(chloroform:tetrahydrofuran:acetic acid = 10:2:1): Rf = 0.59;

NMR: δ = 7.58(1H, dd), 6.18(1H, dd), 5.7-5.3(4H, m), 4.24(2H, s), 4.15-4.03(1H, m), 1.0-0.8(6H, m);

Mass: m/e = 376($M^+$), 358.

(c) (5Z,9Z,13E)-(15S)-1-Hydroxymethyl-15-hydroxy-15-(3-propylcyclopentyl)-16,17,18,19,20-pentanorprosta-5,9,13-triene-1,11-dione [i.e. 2-Decarboxy-2-glycoloyl-15-(3-propylcyclopentyl)-16,17,18,19,20-pentanor-9-deoxy-$\Delta^9$-$PGD_2$]

Starting material: $PGD_2$ compound prepared in Example 1(c);

TLC(chloroform:tetrahydrofuran:acetic acid = 10:2:1): Rf = 0.58;

NMR: δ = 7.58(1H, dd), 6.16(1H, dd), 5.7-5.3(4H, m), 4.22(2H, s), 4.10(1H, m), 1.0-0.8(3H, m);

Mass: m/e = 388($M^+$), 370.

(d) (5Z,9Z,13E)-(15S)-1-Hydroxymethyl-15-hydroxy-16-phenyl-17,18,19,20-tetranorprosta-5,9,13-triene-1,11-dione (i.e. 2-Decarboxy-2-glycoloyl-16-phenyl-17,18,19,20-tetranor-9-deoxy-$\Delta^9$-$PGD_2$)

Starting material: $PGD_2$ compound prepared in Example 1(d);
TLC(chloroform:tetrahydrofuran:acetic acid = 10:2:1):
Rf = 0.56;
NMR: δ = 7.62(1H, dd), 7.5-7.1(5H, m), 6.20(1H, dd), 5.8-5.3(4H, m), 4.25(2H, s);
Mass: m/e = 368($M^+$), 350.

(e) (5Z,9Z,13E)-(15R)-1-Hydroxymethyl-15-hydroxy-16-(3-chlorophenoxy)-17,18,19,20-tetranorprosta-5,9,13-triene-1,11-dione [i.e. 2-Decarboxy-2-glycoloyl-16-(3-chlorophenoxy)-17,18,19,20-tetranor-9-deoxy-$\Delta^9$-$PGD_2$]
Starting material: $PGD_2$ compound prepared in Example 1(e);
TLC(chloroform:tetrahydrofuran:acetic acid = 10:2:1):
Rf = 0.55;
NMR: δ = 7.62(1H, dd), 7.3-6.7(4H, m), 6.20(1H, dd), 5.8-5.3(4H, m), 4.24(2H, s);
Mass: m/e = 418($M^+$), 400.

Example 3

(5Z,9Z,14EZ)-1-Hydroxymethylprosta-5,9,12,14-tetraene-1,11-dione

To a solution of 10 mg of $PGD_2$ compound (prepared in Example 1) in 0.25 ml of tetrahydrofuran, 0.25 ml of a 1N aqueous hydrochloric acid was added, and the resulting solution was refluxed for 20 minutes, cooled and extracted with diethyl ether. The extract was washed with a saturated aqueous solution of sodium chloride, dried, and concentrated under reduced pressure. The residue was purified by column chromatography on silica gel using a mixed solvent of n-hexane and ethyl acetate firstly and ethyl acetate finally as an eluent to give 2 mg of the title compound having the following physical characteristics:
TLC(n-hexane:ethyl acetate = 1:1): Rf = 0.28;

NMR: δ = 7.6-7.4(1H, m), 7.0-6.9(1H, m), 6.5-6.1(3H, m), 5.6-5.25(2H, m), 4.23(2H, bs), 3.7-3.5(1H, m), 0.90(3H, t);

Mass: m/e = 330($M^+$), 259.

The following compounds were obtained by the same procedure as described in Example 3:

(a) (9Z,14EZ)-1-Hydroxymethylprosta-9,12,14-triene-1,11-dione

Starting material: $PGD_1$ compound prepared in Example 1(a);

TLC(n-hexane:ethyl acetate = 1:1): Rf = 0.29;

NMR: δ = 7.47(1H, m), 6.95(1H, d), 6.46-6.15(3H, m), 5.60-5.30(2H, m), 4.20(2H, s), 0.90(3H, t);

Mass: m/e = 332($M^+$).

(b) (5Z,9Z,14EZ)-(17S)-1-Hydroxymethyl-17,20-dimethylprosta-5,9,12,14-tetraene-1,11-dione

Starting material: $PGD_2$ compound prepared in Example 1(b);

TLC(n-hexane:ethyl acetate = 1:1): Rf = 0.30;

NMR: δ = 7.6-7.4(1H, m), 6.95(1H, d), 6.5-6.1(3H, m), 5.6-5.26(2H, m), 4.24(2H, s), 3.60(1H, m), 1.0-0.8(6H, m);

Mass: m/e = 358($M^+$).

(c) (5Z,9Z,14EZ)-1-Hydroxymethyl-15-(3-propylcyclopentyl)-16,17,18,19,20-pentanorprosta-5,9,12,14-tetraene-1,11-dione

Starting material: $PGD_2$ compound prepared in Example 1(c);

TLC(n-hexane:ethyl acetate = 1:1): Rf = 0.30;

NMR: δ = 7.55-7.4(1H, m), 6.97(1H, d), 6.5-6.2(3H, m), 5.6-5.2(2H, m), 4.22(2H, s), 1.0-0.7(3H, m);

Mass: m/e = 370($M^+$).

(d) (5Z,9Z,14EZ)-1-Hydroxymethyl-16-phenyl-17,18,19,20-tetranorprosta-5,9,12,14-tetraene-1,11-dione

Starting material: $PGD_2$ compound prepared in Example 1(d);

TLC(n-hexane:ethyl acetate = 1:1): Rf = 0.27;

NMR: δ = 7.55-7.10(6H, m), 7.00(1H, d), 6.6-6.1(3H, m), 5.6-5.15(2H, m), 4.24(2H, s);

Mass: m/e = 350($M^+$).

(e) (5Z,9Z,14EZ)-1-Hydroxymethyl-16-(3-chlorophenoxy)-17,18,19,20-tetranorprosta-5,9,12,14-tetraene-1,11-dione

Starting material: $PGD_2$ compound prepared in Example 1(e);

TLC(n-hexane:ethyl acetate = 1:1): Rf = 0.25;

NMR: δ = 7.65-7.42(1H, m), 7.3-6.7(5H, m), 6.5-6.1(3H, m), 5.6-5.2 (2H, m), 4.24(2H, s),

Mass: m/e = 400($M^+$).

Reference Example 7

(5Z,13E)-(9α,11α,15S)-9,15-Bis(tetrahydropyran-2-yloxy)-11-hydroxyprosta-5,13-dienoic acid

To a solution of 500 mg of 11-benzoyloxy compound (prepared in Reference Example 3) in 4.8 ml of methanol, 1.6 ml of a 2N aqueous solution of potassium hydroxide was added, and the resulting solution was stirred for 1.5 hours at 50°C, cooled to 5°C and adjusted to pH 2 with 1N aqueous hydrochloric acid. To the reaction mixture, 100 ml of ice-water was added, and the resulting mixture was extracted with ethyl acetate. The extract was washed with water and a saturated aqueous solution of sodium chloride, dried over anhydrous magnesium sulfate and concentrated under reduced pressure to give 483 mg of the crude title compound having the following physical characteristic.

The product obtained was used in the next reaction without purification.

TLC(benzene:ethyl acetate = 2:1): Rf = 0.07.

The following compounds were obtained by the same procedure as described in Reference Example 7:

(a) (13E)-(9α,11α,15S)-9,15-Bis(tetrahydropyran-2-yloxy)-11-hydroxyprost-13-enoic acid

Starting material: 11-benzoyloxy compound prepared in Reference Example 3(a);

TLC(benzene:ethyl acetate = 2:1): Rf = 0.08.

(b) (5Z,13E)-(9α,11α,15S,17S)-9,15-Bis(tetrahydropyran-2-yloxy)-11-hydroxy-17,20-dimethylprosta-5,13-dienoic acid

Starting material: 11-benzoyloxy compound prepared in Reference Example 3(b);

TLC(benzene:ethyl acetate = 2:1): Rf = 0.07.

(c) (5Z,13E)-(9α,11α,15S)-9,15-Bis(tetrahydropyran-2-yloxy)-11-hydroxy-15-(3-propylcyclopentyl)-16,17,18,19,20-pentanorprosta-5,13-dienoic acid

Starting material: 11-benzoyloxy compound prepared in Reference Example 3(c);

TLC(benzene:ethyl acetate = 2:1): Rf = 0.07.

(d) (5Z,13E)-(9α,11α,15S)-9,15-Bis(tetrahydropyran-2-yloxy)-11-hydroxy-16-phenyl-17,18,19,20-tetranorprosta-5,13-dienoic acid

Starting material: 11-benzoyloxy compound prepared in Reference Example 3(d);

TLC(benzene:ethyl acetate = 2:1): Rf = 0.08.

(e) (5Z,13E)-(9α,11α,15R)-9,15-Bis(tetrahydropyran-2-yloxy)-11-hydroxy-16-(3-chlorophenoxy)-17,18,19,20-tetranorprosta-5,13-dienoic acid

Starting material: 11-benzoyloxy compound prepared in Reference Example 3(e);

TLC(benzene:ethyl acetate = 2:1): Rf = 0.06.

Reference Example 8

(5Z,13E)-(9α,11α,15S)-N-Methanesulfonyl-9,15-bis(tetrahydropyran-2-yloxy)-11-hydroxyprosta-5,13-dien -1-amide

To a solution of 407 mg of carboxylic acid (prepared in Reference Example 7) in 4.7 ml of N,N-dimethylformamide, 0.218 ml of triethylamine was added at room temperature, and the solution was cooled to 5°C. To the resulting solution, 0.202 ml of isobutyl chloroformate was added dropwise followed by stirring for 30 minutes at the same temperature and 3.95 ml of a suspension of sodium salt of methylsulfonamide in dimethylsulfoxide (prepared by the procedure hereinafter described) was added and then 1.2 ml of hexamethylphosphoramide was added, and the resulting mixture was stirred for one hour at 2 - 5°C. After the completion of the reaction, 4.3 ml of 1N aqueous hydrochloric acid was added with ice-water cooling and 100 ml of ice-water was added to the reaction mixture. The resulting mixture was extracted with ethyl acetate, and the extract was washed with water and a saturated aqueous solution of sodium chloride, dried over anhydrous magnesium sulfate, and concentrated under reduced pressure. The residue was purified by column chromatography on silica gel using a mixed solvent of n-hexane and ethyl acetate firstly and ethyl acetate finally as an eluent to give 308 mg of the title compound having the following physical characteristics:

TLC(ethyl acetate): Rf = 0.57;

NMR: δ = 5.6-5.2(4H, m), 4.8-4.5(2H, m), 4.4-3.0(9H, m), 3.28(3H, s);

IR : ν = 3650-2600, 2930, 2850, 1710 $cm^{-1}$;

Mass: m/e = 413, 395, 341.

The suspension of sodium salt of methylsulfonamide in dimethylsulfoxide used in the hereinbefore described reaction was prepared as follows:

To 3.9 ml of dry dimethylsulfoxide, 295 mg of sodium hydride was added, and the resulting mixture was stirred for one hour at 75°C. To a solution of 370 mg of methanesulfonamide in 2 ml of dry dimethylsulfoxide, 1.95 ml of a solution of sodiomethylsulfinylcarbanion in dimethylsulfoxide, prepared by the above reaction, was added dropwise with water cooling, and the reaction mixture was stirred for one hour at 25°C to give the suspension of sodium salt of methylsulfonamide in dimethylsulfoxide.

The following compounds were obtained by the same procedure as described in Reference Example 8:

(a) (13E)-(9α,11α,15S)-N-Methanesulfonyl-9,15-bis(tetrahydropyran-2-yloxy)-11-hydroxyprost-13-en -1-amide

Starting material: carboxylic acid prepared in Reference Example 7(a);

TLC(ethyl acetate): Rf = 0.57;

NMR: δ = 5.6-5.3(2H, m), 4.8-4.5(2H, m), 3.27(3H, s), 0.88(3H, t);

IR : ν = 2930, 1710, 1446, 1340 $cm^{-1}$;

Mass: m/e = 415, 397.

(b) (5Z,13E)-(9α,11α,15S,17S)-N-Methanesulfonyl-9,15-bis(tetrahydropyran-2-yloxy)-11-hydroxy-17,20-dimethyl-prosta-5,13-dien -1-amide

Starting material: carboxylic acid prepared in Reference Example 7(b);

TLC(ethyl acetate): Rf = 0.58;

NMR: δ = 5.6-5.17(4H, m), 4.8-4.5(2H, m), 3.3(3H, s), 1.0-0.7(6H, m);

IR : ν = 2940, 1710, 1445, 1340 $cm^{-1}$;

Mass: m/e = 441, 423.

(c) (5Z,13E)-(9α,11α,15S)-N-Methanesulfonyl-9,15-bis(tetrahydropyran-2-yloxy)-11-hydroxy-15-(3-propylcyclopentyl)-16,17,18,19,20-pentanorprosta-5,13-dien -1-amide

Starting material: carboxylic acid prepared in Reference Example 7(c);

TLC(ethyl acetate): Rf = 0.58;

NMR: δ = 5.6-5.2(4H, m), 4.8-4.5(2H, m), 3.30(3H, s), 0.88(3H, t);

IR : ν = 2930, 1710 $cm^{-1}$;

Mass: m/e = 453, 435.

(d) (5Z,13E)-(9α,11α,15S)-N-Methanesulfonyl-9,15-bis(tetrahydropyran-2-yloxy)-11-hydroxy-16-phenyl-17,18,19,20-tetranor-prosta-5,13-dien -1-amide

Starting material: carboxylic acid prepared in Reference Example 7(d);

TLC(ethyl acetate): Rf = 0.55;

NMR: δ = 7.5-7.1(5H, m), 57-5.2(4H, m), 4.8-4.5(2H, m), 3.30(3H, s);

IR : ν = 2930, 1710 $cm^{-1}$;

Mass: m/e = 433, 415.

(e) (5Z,13E)-(9α,11α,15R)-N-Methanesulfonyl-9,15-bis(tetrahydropyran-2-yloxy)-11-hydroxy-16-(3-chlorophenoxy)-17,18,19,20-tetranorprosta-5,13-dien -1-amide

Starting material: carboxylic acid prepared in Reference Example 7(e);

TLC(ethyl acetate): Rf = 0.56;

NMR: $\delta$ = 7.3-6.7(4H, m), 5.6-5.2(4H, m), 4.8-4.5(2H, m), 3.30(3H, s);

IR : $\nu$ = 2930, 1710, 1600 $cm^{-1}$;

Mass: m/e = 483, 465.

Reference Example 9

(5Z,13E)-(9α,15S)-N-Methanesulfonyl-9,15-bis(tetrahydropyran-2-yloxy)-11-oxoprosta-5,13-dien -1-amide

With cooling to -30°C, 0.64 ml of Jones' reagent (prepared by addition of 23 ml of sulfuric acid to a solution of 26.7 g of chromium trioxide in 40 ml of water followed by addition of water to make the total volume 100 ml) was added dropwise to a solution of 342 mg of 11-hydroxy compound(prepared in Reference Example 8) in 3 ml of acetone, and the resulting solution was stirred for 20 minutes at the same temperature. To the reaction mixture, 0.26 ml of isopropanol was added dropwise at that temperature followed by stirring for 20 minutes to quench the excess chromium trioxide. After addition of 50 ml of ice-water, the reaction mixture was extracted with ethyl acetate. The extract was washed with water and a saturated aqueous solution of sodium chloride, dried over magnesium sulfate and concentrated under reduced pressure. The residue was purified by column chromatography on silica gel using a mixed solvent of n-hexane and ethyl acetate firstly and ethyl acetate

finally as eluent to give 164 mg of the title compound having the following physical characteristics:

TLC(n-hexane:ethyl acetate = 1:2): Rf = 0.49;

NMR: δ = 5.7-5.2(4H, m), 4.8-4.5(2H, m), 4.4-3.0(6H, m), 3.28(3H, s);

IR : ν = 3300-3000, 2930, 1740, 1720 $cm^{-1}$;

Mass: m/e = 411, 393, 340, 322.

The following compounds were obtained by the same procedure as described in Reference Example 9:

(a) (13E)-(9α,15S)-N-Methanesulfonyl-9,15-bis(tetrahydropyran-2-yloxy)-11-oxoprost-13-en -1-amide

Starting material: 11-hydroxy compound prepared in Reference Example 8(a);

TLC(n-hexane-ethyl acetate = 1:2): Rf = 0.49;

NMR: δ = 5.7-5.3(2H, m), 4.8-4.5(2H, m), 3.27(3H, s), 0.88(3H, t);

IR : ν = 2930, 1740, 1720, 1440, 1340 $cm^{-1}$;

Mass: m/e = 413, 395.

(b) (5Z,13E)-(9α,15S,17S)-N-Methanesulfonyl-9,15-bis(tetrahydropyran-2-yloxy)-11-oxo-17,20-dimethylprosta-5,13-dien -1-amide

Starting material: 11-hydroxy compound prepared in Reference Example 8(b);

TLC(n-hexane:ethyl acetate = 1:2): Rf = 0.51;

NMR: δ = 5.9-5.2(4H, m), 4.8-4.4(2H, m), 3.26(3H, s), 1.0-0.7(6H, m);

IR : ν = 2935, 1742, 1720, 1116, 1020 $cm^{-1}$;

Mass: m/e = 439, 421.

(c) (5Z,13E)-(9α,15S)-N-Methanesulfonyl-9,15-bis(tetrahydropyran-2-yloxy)-11-oxo-15-(3-propylcyclopentyl)-16,17,18,19,20-pentanorprosta-5,13-dien -1-amide

Starting material: 11-hydroxy compound prepared in Reference Example 8(c);

TLC(n-hexane:ethyl acetate = 1:2): Rf = 0.50;

NMR: δ = 5.9-5.2(4H, m), 4.8-4.4(2H, m), 3.3(3H,s), 0.87(3H, t);

IR : ν = 2930, 1740, 1720 $cm^{-1}$;

Mass: m/e = 451, 333.

(d) (5Z,13E)-(9α,15S)-N-Methanesulfonyl-9,15-bis(tetrahydropyran-2-yloxy)-11-oxo-16-phenyl-17,18,19,20-tetranorprosta-5,13-dien -1-amide

Starting material: 11-hydroxy compound prepared in Reference Example 8(d);

TLC(n-hexane:ethyl acetate = 1:2): Rf = 0.47;

NMR: δ = 7.5-7.1(5H, m), 5.7-5.2(4H, m), 4.8-4.5(2H, m), 3.30(3H, s);

IR : ν = 2930, 1740, 1716 $cm^{-1}$;

Mass: m/e = 431, 413.

(e) (5Z,13E)-(9α,15R)-N-Methanesulfonyl-9,15-bis(tetrahydropyran-2-yloxy)-11-oxo-16-(3-chlorophenoxy)-17,18,19,20-tetranorprosta-5,13-dien -1-amide

Starting material: 11-hydroxy compound prepared in Reference Example 8(e);

TLC(n-hexane:ethyl acetate = 1:2): Rf = 0.48;

NMR: δ = 7.3-6.7(4H, m), 5.7-5.2(4H, m), 4.8-4.5(2H, m), 3.3(3H, s);

IR : ν = 2930, 1740, 1720, 1600 $cm^{-1}$;

Mass: m/e = 481, 463.

Reference Example 10

(5Z,13E)-(9α,15S)-N-Methanesulfonyl-9,15-dihydroxy-11-oxoprosta-5,13-dien -1-amide (i.e. $PGD_2$ methanesulfonylamide)

To a solution of 196 mg of 9,15-bis(tetrahydropyran-2-yloxy) compound (prepared in Reference Example 9) in 0.32 ml of tetrahydrofuran, 3.2 ml of 65% aqueous acetic acid was added, and the resulting solution was stirred for 8 minutes at 80°C followed by addition of 50 ml of ice-water and then extracted with ethyl acetate. The extract was washed with water and a saturated aqueous solution of sodium chloride, dried over anhydrous magnesium sulfate, and concentrated with addition of toluene under reduced pressure at low temperature. The residue was purified by column chromatography on silica gel using a mixed solvent of n-hexane and ethyl acetate firstly and ethyl acetate finally as eluent to give 42 mg of the title compound having the following physical characteristics:

Melting point: 132°C;

TLC(chloroform:tetrahydrofuran:acetic acid = 10:2:1): Rf = 0.14;

NMR(a mixed solution of acetone-$d_6$ and $CDCl_3$):

δ = 5.70-5.33(4H, m), 4.48(1H, m), 4.12(1H, m), 3.25(3H, m), 0.88(3H, t);

IR(KBr method): ν = 3650-3200, 2930, 1740, 1720 $cm^{-1}$;

Mass: m/e = 411, 393, 340, 322.

The following compounds were obtained by the same procedure as described in Reference Example 10:

(a) (13E)-(9α,15S)-N-Methanesulfonyl-9,15-dihydroxy-11-oxo-prost-13-en -1-amide (i.e. $PGD_1$ methanesulfonylamide)

Starting material: 9,15-bis(tetrahydropyran-2-yloxy) compound prepared in Reference Example 9(a);

TLC(chloroform:tetrahydrofuran:acetic acid = 10:2:1):

Rf = 0.14;

NMR(a mixed solution of acetone-$d_6$ and $CDCl_3$):

δ = 5.6-5.4(2H, m), 4.46(1H, m), 4.08(1H, m), 3.26(3H, s), 0.88(3H, t);

IR : ν = 3420, 2930, 1740, 1720, 1460, 1320, 1118 $cm^{-1}$;

Mass: m/e = 413, 395.

(b) (5Z,13E)-(9α,15S,17S)-N-Methanesulfonyl-9,15-dihydroxy-11-oxo-17,20-dimethylprosta-5,13-dien -1-amide (i.e. 17S,20-Dimethyl-$PGD_2$ methanesulfonylamide)

Starting material: 9,15-bis(tetrahydropyran-2-yloxy) compound prepared in Reference Example 9(b);

TLC(chloroform:tetrahydrofuran:acetic acid = 10:2:1):

Rf = 0.16;

NMR(a mixed solution of acetone-$d_6$ and $CDCl_3$):

δ = 5.7-5.3(4H, m), 4.46(1H, m), 4.10(1H, m), 3.24(3H, s), 1.0-0.7(6H, m);

IR : ν = 3420, 3230, 2930, 1740, 1718, 1320, 1120 $cm^{-1}$;

Mass: m/e = 439, 421.

(c) (5Z,13E)-(9α,15S)-N-Methanesulfonyl-9,15-dihydroxy-11-oxo-15-(3-propylcyclopentyl)-16,17,18,19,20-pentanor-prosta-5,13-dien -1-amide [i.e. 15-(3-Propylcyclopentyl)-16,17,18,19,20-pentanor-$PGD_2$ methanesulfonylamide]

Starting material: 9,15-bis(tetrahydropyran-2-yloxy) compound prepared in Reference Example 9(c)

TLC(chloroform:tetrahydrofuran:acetic acid = 10:2:1):

Rf = 0.16;

NMR(a mixed solution of acetone-$d_6$ and $CDCl_3$):

δ = 5.7-5.3(4H, m), 4.48(1H, d), 4.13(1H, m), 3.25(3H, s), 0.88(3H, t);

IR : ν = 3420, 2930, 1740, 1720 $cm^{-1}$;

Mass: m/e = 451, 433.

(d) (5Z,13E)-(9α,15S)-N-Methanesulfonyl-9,15-dihydroxy-11-oxo-16-phenyl-17,18,19,20-tetranorprosta-5,13-dien -1-amide (i.e. 16-Phenyl-17,18,19,20-tetranor-$PGD_2$ methanesulfonylamide)

Starting material: 9,15-bis(tetrahydropyran-2-yloxy) compound prepared in Reference Example 9(d);

TLC(chloroform:tetrahydrofuran:acetic acid = 10:2:1):

Rf = 0.13;

NMR(a mixed solution of acetone-$d_6$ and $CDCl_3$):

δ = 7.5-7.1(5H, m), 5.7-5.4(4H, m), 4.47(1H, m), 4.13(1H, m), 3.26(3H, s);

IR : ν = 3420, 2930, 1740, 1720 $cm^{-1}$;

Mass: m/e = 431, 413.

(e) (5Z,13E)-(9α,15R)-N-Methanesulfonyl-9,15-dihydroxy-11-oxo-16-(3-chlorophenoxy)-17,18,19,20-tetranorprosta-5,13-dien -1-amide [i.e. 16-(3-Chlorophenoxy)-17,18,19,20-tetranor-$PGD_2$ methanesulfonylamide)

Starting material: 9,15-bis(tetrahydropyran-2-yloxy) compound prepared in Reference Example 9(e);

TLC(chloroform:tetrahydrofuran:acetic acid = 10:2:1):

Rf = 0.13;

NMR(a mixed solution of acetone-$d_6$ and $CDCl_3$):

$\delta$ = 7.3-6.7(4H, m), 5.70-5.30(4H, m), 4.6-4.3(2H, m), 3.94(2H, d), 3.30(3H, s);

IR : $\nu$ = 3420, 2930, 1740, 1720, 1600 $cm^{-1}$;

Mass: m/e = 481, 463.

Example 4

(5Z,9Z,13E)-(15S)-N-Methanesulfonyl-11-oxo-15-hydroxyprosta-5,9,13-trien -1-amide (i.e. 9-Deoxy-$\Delta^9$-$PGD_2$ methanesulfonylamide)

To a solution of 50 mg of $PGD_2$ methanesulfonylamide (prepared in Reference Example 10) in 1 ml of ethanol, 58 ml of Tris-hydrochloric acid buffer solution (prepared by the same procedure described in Example 2) (pH 7.2) was added with stirring, and the resulting solution was stirred for 30 minutes at 37°C. Cooling with ice-water, the reaction mixture was adjusted with 1N aqueous hydrochloric acid to pH 4, and extracted with ethyl acetate. The extract was washed with water and a saturated aqueous solution of sodium chloride, dried over anhydrous magnesium sulfate, and concentrated under reduced pressure. The residue was purified by column chromatography on silica gel using a mixed solvent of n-hexane and ethyl acetate firstly and ethyl acetate finally as eluent to give 8.4 mg of the title compound having the following physical characteristics:

TLC(chloroform:tetrahydrofuran:acetic acid = 10:2:1): Rf = 0.42;

NMR: $\delta$ = 7.6(1H, dd), 6.18(1H, dd), 5.7-5.62(2H, m), 5.6-5.3(2H, m), 4.22-4.1(1H, m), 3.28(3H, s), 2.94-2.8(1H, m), 2.74-2.64(1H, m);

IR : $\nu$ = 3650-2600, 2930, 1700, 1620 $cm^{-1}$;

Mass: m/e = 411($M^+$), 393, 322.

The following compounds were obtained by the same procedure as described in Example 4:

(a) (9Z,13E)-(15S)-N-Methanesulfonyl-11-oxo-15-hydroxyprosta-9,13-dien -1-amide (i.e. 9-Deoxy-$\Delta^9$-$PGD_1$ methanesulfonylamide)

Starting material: $PGD_1$ methanesulfonylamide compound prepared in Reference Example 10(a);

TLC(chloroform:tetrahydrofuran:acetic acid = 10:2:1): Rf = 0.42;

NMR: $\delta$ = 7.60(1H, dd), 6.18(1H, dd), 5.66(2H, m), 4.14(1H, m), 3.28(3H, s), 0.88(3H, t);

IR : $\nu$ = 3420, 2930, 1700, 1330 $cm^{-1}$;

Mass: m/e = 413($M^+$), 395.

(b) (5Z,9Z,13E)-(15S,17S)-N-Methanesulfonyl-11-oxo-15-hydroxy-17,20-dimethylprosta-5,9,13-trien -1-amide (i.e. 17S,20-Dimethyl-9-deoxy-$\Delta^9$-$PGD_2$ methanesulfonylamide)

Starting material: $PGD_2$ methanesulfonylamide compound prepared in Reference Example 10(b);

TLC(chloroform:tetrahydrofuran:acetic acid = 10:2:1): Rf = 0.44;

NMR: $\delta$ = 7.60(1H, dd), 6.17(1H, dd), 5.66(2H, m), 5.6-5.3(2H, m), 4.16(1H, m), 3.28(3H, s), 1.0-0.8(6H, m);

IR : $\nu$ = 2930, 1700, 1330, 1120 $cm^{-1}$;

Mass: m/e = 439($M^+$), 421.

(c) (5Z,9Z,13E)-(15S)-N-Methanesulfonyl-11-oxo-15-hydroxy-15-(3-propylcyclopentyl)-16,17,18,19,20-pentanorprosta-5,9,13-trien -1-amide [i.e. 15-(3-Propylcyclopentyl)-16,17,18,19,20-pentanor-9-deoxy-$\Delta^9$-$PGD_2$ methanesulfonylamide]

Starting material: $PGD_2$ methanesulfonylamide compound prepared in Reference Example 10(c);

TLC(chloroform:tetrahydrofuran:acetic acid = 10:2:1): Rf = 0.43;

NMR: $\delta$ = 7.61(1H, dd), 6.18(1H, dd), 5.68(2H, m), 5.6-5.3(2H, m), 4.20(1H, m), 3.28(3H, s), 0.88(3H, t);

IR : $\nu$ = 3420, 2930, 1705 $cm^{-1}$;

Mass: m/e = 451($M^+$), 433.

(d) (5Z,9Z,13E)-(15S)-N-Methanesulfonyl-11-oxo-15-hydroxy-16-phenyl-17,18,19,20-tetranorprosta-5,9,13-trien -1-amide (i.e. 16-Phenyl-17,18,19,20-tetranor-9-deoxy-$\Delta^9$-$PGD_2$ methanesulfonylamide)

Starting material: $PGD_2$ methanesulfonylamide compound prepared in Reference Example 10(d);

TLC(chloroform:tetrahydrofuran:acetic acid = 10:2:1): Rf = 0.41;

NMR: $\delta$ = 7.51(1H, dd), 7.5-7.1(5H, m), 6.18(1H, dd), 5.65(2H, m), 5.6-5.3(2H, m), 4.25(1H, m), 3.30(3H, s);

IR : $\nu$ = 2930, 1703 $cm^{-1}$;

NMR: m/e = 431($M^+$), 413.

(e) (5Z,9Z,13E)-(15R)-N-Methanesulfonyl-11-oxo-15-hydroxy-16-(3-chlorophenoxy)-17,18,19,20-tetranorprosta-5,9,13-trien -1-amide [i.e. 16-(3-Chlorophenoxy)-17,18,19,20-tetranor-9-deoxy-$\Delta^9$-$PGD_2$ methanesulfonylamide]

Starting material: $PGD_2$ methanesulfonylamide compound prepared in Reference Example 10(e);

TLC(chloroform:tetrahydrofuran:acetic acid = 10:2:1): Rf = 0.41;

NMR: $\delta$ = 7.60(1H, dd), 7.3-6.7(4H, m), 6.20(1H, dd), 5.70(2H, m), 5.50-5.30(2H, m), 4.20(1H, m), 3.30(3H, s);

IR : $\nu$ = 2930, 1700, 1600 $cm^{-1}$;

Mass: m/e = 481($M^+$), 463.

Example 5

(5Z,9Z,14EZ)-N-Methanesulfonyl-11-oxoprosta-5,9,12,14-tetraen -1-amide

To a solution of 22 mg of $PGD_2$ methanesulfonylamide (prepared in Reference Example 10) in 0.5 ml of tetrahydrofuran, 0.5 ml of a 1N aqueous hydrochloric acid was added, and the resulting solution was refluxed for 60 minutes. To the resulting mixture, 50 ml of ice-water was added and the resulting mixture was extracted with diethyl ether.
The extract was washed with water and a saturated aqueous solution of sodium chloride, dried over anhydrous magnesium sulfate, and concentrated under reduced pressure. The residue was purified by column chromatography on silica gel using a mixed solvent of n-hexane and ethyl acetate as eluent to give 8 mg of the title compound having the following physical

characteristics:

TLC(chloroform:tetrahydrofuran:acetic acid = 10:2:1): Rf = 0.70;

NMR: δ = 8.9-8.8(1H, m), 7.5(1H, dd), 6.95(1H, bd), 6.39(1H, dd), 6.5-6.2(2H, m), 5.5-5.3(2H, m), 3.7-3.6(1H, m), 3.29(3H, s);

IR : ν = 3650-3000, 2925, 1710, 1680 $cm^{-1}$;

Mass: m/e = 393($M^+$), 322.

The following compounds were obtained by the same procedure as described in Example 5:

(a) (9Z,14EZ)-N-Methanesulfonyl-11-oxoprosta-9,12,14-trien-1-amide

Starting material: $PGD_1$ methanesulfonylamide compound prepared in Reference Example 10(a);

TLC(chloroform:tetrahydrofuran:acetic acid = 10:2:1): Rf = 0.70;

NMR: δ = 7.50(1H, dd), 6.96(1H, d), 6.46-6.25(3H, m), 3.65(1H, m), 3.28(3H, s), 090(3H, t);

IR : ν = 2920, 1710, 1680, 1620 $cm^{-1}$;

Mass: m/e = 395($M^+$).

(b) (5Z,9Z,14EZ)-(17S)-N-Methanesulfonyl-11-oxo-17,20-dimethylprosta-5,9,12,14-tetraen -1-amide

Starting material: $PGD_2$ methanesulfonylamide compound prepared in Reference Example 10(b);

TLC(chloroform:tetrahydrofuran:acetic acid = 10:2:1): Rf = 0.72;

NMR: δ = 7.51(1H, dd), 6.96(1H, dd), 6.50-6.23(3H, m), 5.50-5.23(2H, m), 3.66(1H, m), 3.30(3H, s), 1.0-0.8(6H, m);

IR : ν = 2930, 1710, 1680, 1622 $cm^{-1}$;

Mass: m/e = 421($M^+$).

(c) (5Z,9Z,14EZ)-N-Methanesulfonyl-11-oxo-15-(3-propylcyclopentyl)-16,17,18,19,20-pentanorprosta-5,9,12,14-tetraen-1-amide

Starting material: $PGD_2$ methanesulfonylamide compound prepared in Reference Example 10(c);

TLC(chloroform:tetrahydrofuran:acetic acid = 10:2:1): Rf = 0.72;

NMR: δ = 7.51(1H, dd), 6.96(1H, dd), 6.50-6.23(3H, m), 5.5-5.2(2H, m), 3.30(3H, s), 0.90(3H, t);

IR : ν = 2930, 1710, 1680, 1622 $cm^{-1}$;

Mass: m/e = 433($M^+$).

(d) (5Z,9Z,14EZ)-N-Methanesulfonyl-11-oxo-16-phenyl-17,18,19,20-tetranorprosta-5,9,12,14-tetraen -1-amide

Starting material: $PGD_2$ methanesulfonylamide compound prepared in Reference Example 10(d);

TLC(chloroform:tetrahydrofuran:acetic acid = 10:2:1): Rf = 0.68;

NMR: δ = 7.5-7.1(6H, m), 6.92(1H, d), 6.45-6.20(3H, m), 5.50-5.23(2H, m), 3.70(1H, m), 3.30(3H, s);

IR : ν = 2930, 1710, 1680, 1620 $cm^{-1}$;

Mass: m/e = 413($M^+$).

(e) (5Z,9Z,14EZ)-N-Methanesulfonyl-11-oxo-16-(3-chlorophenoxy)-17,18,19,20-tetranorprosta-5,9,12,14-tetraen -1-amide

Starting material: $PGD_2$ methanesulfonylamide compound prepared in Reference Example 10(e);

TLC(chloroform:tetrahydrofuran:acetic acid = 10:2:1): Rf = 0.68;

NMR: $\delta$ = 7.52(1H, dd), 7.3-6.7(5H, m), 6.5-6.2(3H, m), 5.4-5.2(2H, m), 3.68(1H, m), 3.30(3H, s);

IR : $\nu$ = 2930, 1710, 1680, 1620, 1600 $cm^{-1}$;

Mass: m/e = 463($M^+$).

Reference Example 11

(5Z,13E)-(9α,11α,15S)-N,N-Dimethyl-9,15-bis(tetrahydropyran-2-yloxy)-11-hydroxyprosta-5,13-dien -1-amide

Under an atmosphere of nitrogen, 0.32 ml of triethylamine was added to a solution of 507 mg of carboxylic acid (prepared in Reference Example 7) in 20 ml of methylene chloride at 0°C and the resulting solution was stirred. To the resulting solution, 0.27 ml of isobutyl chloroformate was added dropwise at the same temperature, and the resulting mixture was stirred for 30 minutes and warmed to room temperature. Gaseous dimethylamine was blown through the resulting mixture for one hour, and the reaction mixture was stirred for one hour, neutralized with a aqueous solution of oxalic acid and extracted with ethyl acetate. The extract was washed with a saturated aqueous solution of sodium chloride, dried, and concentrated under reduced pressure. The residue was purified by column chromatography on silica gel using a mixed solvent of n-hexane and ethyl acetate firstly and ethyl acetate finally as eluent to give 522 mg of the title compound having the following physical characteristics:

TLC(ethyl acetate): Rf = 0.21;

NMR: $\delta$ = 5.7-5.2(4H, m), 4.8-4.5(2H, m), 4.3-3.2(7H, m), 2.96(6H, bs), 1.0-0.7(3H, m);

IR : $\nu$ = 3450, 2930, 1640 $cm^{-1}$;

Mass: m/e = 464, 447.

The following compounds were obtained by the same procedure as described in Reference Example 11:

(a) (13E)-(9α,11α,15S)-N,N-Dimethyl-9,15-bis(tetrahydropyran-2-yloxy)-11-hydroxyprost-13-en -1-amide

Starting material: carboxylic acid prepared in Reference Example 7(a);

TLC(ethyl acetate): Rf = 0.22;

NMR: δ = 5.6-5.4(2H, m), 4.8-4.5(2H, m), 2.94(6H, s), 0.86(3H, t);

IR : ν = 3460, 2940, 1642 $cm^{-1}$;

Mass: m/e = 449.

(b) (5Z,13E)-(9α,11α,15S,17S)-N,N-Dimethyl-9,15-bis(tetrahydropyran-2-yloxy)-11-hydroxy-17,20-dimethylprosta-5,13-dien-1-amide

Starting material: carboxylic acid prepared in Reference Example 7(b);

TLC(ethyl acetate): Rf = 0.22;

NMR: δ = 5.7-5.3(4H, m), 4.8-4.5(2H, m), 2.95(6H, s), 1.0-0.8(6H, m);

IR : ν = 3450, 2930, 1700, 1640 $cm^{-1}$;

Mass: m/e = 475.

(c) (5Z,13E)-(9α,11α,15S)-N,N-Dimethyl-9,15-bis(tetrahydropyran-2-yloxy)-11-hydroxy-15-(3-propylcyclopentyl)-16,17,18,19,20-pentanorprosta-5,13-dien -1-amide

Starting material: carboxylic acid prepared in Reference Example 7(c);

TLC(ethyl acetate): Rf = 0.23;

NMR: δ = 5.6-5.2(4H, m), 4.8-4.5(2H, m), 2.95(6H, s), 0.87(3H, t);

IR : ν = 3450, 2940, 1640 $cm^{-1}$;

Mass: m/e = 487.

(d) (5Z,13E)-(9α,11α,15S)-N,N-Dimethyl-9,15-bis(tetrahydropyran-2-yloxy)-11-hydroxy-16-phenyl-17,18,19,20-tetranorprosta-5,13-dien -1-amide

Starting material: carboxylic acid prepared in Reference Example 7(d);

TLC(ethyl acetate): Rf = 0.20;

NMR: δ = 7.5-7.1(5H, m), 5.7-5.2(4H, m), 4.8-4.5(2H, m), 2.96(6H, s);

IR : ν = 3450, 2930, 1640 $cm^{-1}$;

Mass: m/e = 484, 467.

(e) (5Z,13E)-(9α,11α,15R)-N,N-Dimethyl-9,15-bis(tetrahydropyran-2-yloxy)-11-hydroxy-16-(3-chlorophenoxy)-17,18,19,20-tetranorprosta-5,13-dien -1-amide

Starting material: carboxylic acid prepared in Reference Example 7(e);

TLC(ethyl acetate): Rf = 0.20;

NMR: δ = 7.3-6.7(4H, m), 5.7-5.2(4H, m), 4.8-4.5(2H, m), 2.98(6H, s);

IR : ν = 3460, 2930, 1640, 1600 $cm^{-1}$;

Mass: m/e = 517.

Reference Example 12

(5Z,13E)-(9α,15S)-N,N-Dimethyl-9,15-bis(tetrahydropyran-2-yloxy)-11-oxoprosta-5,13-dien -1-amide

Under an atmosphere of nitrogen, a mixture of 1.8 ml of pyridine and 1.1 g of chromium trioxide in 35 ml of methylene

chloride was stirred for 15 minutes. To the reaction mixture, 5 g of Celite was added and 300 mg of 11-hydroxy compound (prepared in Reference Example 11) in 5 ml of methylene chloride was added at 0°C. The mixture obtained was stirred for 30 minutes, diluted with ethyl acetate and filtered. The filtrate was washed with water, a 5% aqueous solution of cupric acetate and a saturated aqueous solution of sodium chloride, dried, and concentrated under reduced pressure. The residue was purified by column chromatography on silica gel using a mixed solvent of n-hexane and ethyl acetate firstly and ethyl acetate finally as eluent to give 289 mg of the title compound having the following physical characteristics:

TLC(ethyl acetate): Rf = 0.37;

NMR: δ = 5.8-5.3(4H, m), 4.8-3.2(8H, m), 2.95(6H, bs), 1.0-0.7(3H, m);

IR : ν = 2930, 1740, 1645, 1125, 1015, 980 $cm^{-1}$;

Mass: m/e = 412, 445, 361.

The following compounds were obtained by the same procedure as described in Reference Example 12:

(a) (13E)-(9α,15S)-N,N-Dimethyl-9,15-bis(tetrahydropyran-2-yloxy)-11-oxoprost-13-en-1-amide

Starting material: 11-hydroxy compound prepared in Reference Example 11(a);

TLC(ethyl acetate): Rf = 0.37;

NMR: δ = 5.7-5.3(2H, m), 4.8-4.5(2H, m), 2.96(6H, s), 0.88(3H, t);

IR : ν = 2930, 1740, 1644 $cm^{-1}$;

Mass: m/e = 447.

(b) (5Z,13E)-(9α,15S,17S)-N,N-Dimethyl-9,15-bis(tetrahydropyran-2-yloxy)-11-oxo-17,20-dimethylprosta-5,13-dien-1-amide

Starting material: 11-hydroxy compound prepared in Reference Example 11(b);

TLC(ethyl acetate): Rf = 0.37;

NMR: δ = 5.8-5.3(4H, m), 4.66(2H, m), 2.96(6H, s), 1.0-0.7(6H, m);

IR : ν = 2930, 1740, 1648 $cm^{-1}$;

Mass: m/e = 490, 473.

(c) (5Z,13E)-(9α,15S)-N,N-Dimethyl-9,15-bis(tetrahydropyran-2-yloxy)-11-oxo-15-(3-propylcyclopentyl)-16,17,18,19,20-pentanorprosta-5,13-dien-1-amide

Starting material: 11-hydroxy compound prepared in Reference Example 11(c);

TLC(ethyl acetate): Rf = 0.38;

NMR: δ = 5.7-5.3(4H, m), 4.8-4.4(2H, m), 2.95(6H, t), 0.86(3H, t);

IR : ν = 2935, 1740, 1648 $cm^{-1}$;

Mass m/e = 502, 485.

(d) (5Z,13E)-(9α,15S)-N,N-Dimethyl-9,15-bis(tetrahydropyran-2-yloxy)-11-oxo-16-phenyl-17,18,19,20-tetranorprosta-5,13-dien-1-amide

Starting material: 11-hydroxy compound prepared in Reference Example 11(d);

TLC(ethyl acetate): Rf = 0.36;

NMR: δ = 7.5-7.1(5H, m), 5.8-5.3(4H, m), 3.00(6H, m);

IR : $\nu$ = 2930, 1740, 1645 $cm^{-1}$;

Mass: m/e = 482, 465.

(e) (5Z,13E)-(9α,15R)-N,N-Dimethyl-9,15-bis(tetrahydropyran-2-yloxy)-11-oxo-16-(3-chlorophenoxy)-17,18,19,20-tetranor-prosta-5,13-dien-1-amide

Starting material: 11-hydroxy compound prepared in Reference Example 11(e);

TLC(ethyl acetate): Rf = 0.36;

NMR: δ = 7.3-6.7(4H, m), 5.8-5.3(4H, m), 2.98(6H, s);

IR : $\nu$ = 2930, 1740, 1650, 1600 $cm^{-1}$;

Mass: m/e = 532, 515.

Example 6

(5Z,13E)-(9α,15S)-N,N-Dimethyl-9,15-dihydroxy-11-oxoprosta-5,13-dien-1-amide (i.e. $PGD_2$ N,N-dimethylamide)

To a solution of 274 mg of 9,15-bis(tetrahydropyran-2-yloxy) compound (prepared in Reference Example 12) in 1 ml of tetrahydrofuran, 10 ml of 65% (v/v) aqueous acetic acid was added, and the resulting mixture was stirred for 10 minutes at 70°C. After cooling, the reaction mixture was diluted with ice-water and extracted with ethyl acetate. The extract was washed with ice-water and a saturated aqueous solution of sodium chloride, dried, and concentrated with addition of dry toluene under reduced pressure at low temperature. The residue was purified by column chromatography on silica gel using a mixed solvent of n-hexane and ethyl acetate firstly, ethyl acetate secondly and a mixed solvent of ethyl acetate and acetone finally as an eluent to give 148 mg of the title

compound having the following physical characteristics:

TLC(chloroform:tetrahydrofuran:acetic acid = 10:2:1): Rf = 0.17

NMR: δ = 5.75-5.35(4H, m), 4.52-4.42(1H, m), 4.18-4.04(1H, m), 3.02(3H, s), 2.95(3H, s), 3.0-2.82(1H, m), 1.0-0.8(3H, m);

IR : ν = 3400, 2925, 1735, 1620 $cm^{-1}$;

Mass: m/e = 379($M^+$), 361, 343.

The following compounds were obtained by the same procedure as described in Example 6:

(a) (13E)-(9α,15S)-N,N-Dimethyl-9,15-dihydroxy-11-oxoprost-13-en-1-amide (i.e. $PGD_1$ N,N-dimethylamide).

Starting material: 9,15-bis(tetrahydropyran-2-yloxy) compound prepared in Reference Example 12(a);

TLC(chloroform:tetrahydrofuran:acetic acid = 10:2:1): Rf = 0.17;

NMR(a mixed solution of acetone-$d_6$ and $CDCl_3$): δ = 5.7-5.4(2H, m), 4.46(1H, m), 4.10(1H, m), 3.01(3H, s), 2.94(3H, s), 0.88(3H, t);

IR : ν = 3400, 2920, 1738, 1622 $cm^{-1}$;

Mass: m/e = 381($M^+$), 363, 345.

(b) (5Z,13E)-(9α,15S,17S)-N,N-Dimethyl-9,15-dihydroxy-11-oxo-17,20-dimethylprosta-5,13-dien-1-amide (i.e. 17S,20-Dimethyl-$PGD_2$ N,N-dimethylamide)

Starting material: 9,15-bis(tetrahydropyran-2-yloxy) compound prepared in Reference Example 12(b);

TLC(chloroform:tetrahydrofuran:acetic acid = 10:2:1): Rf = 0.17;

NMR: δ = 5.7-5.4(4H, m), 4.46(1H, m), 4.10(1H, m), 3.02(3H, s), 2.95(3H, s), 1.0-0.8(6H, m);

IR : ν = 3400, 2930, 1735, 1625 $cm^{-1}$;

Mass: m/e = 407, 389, 371.

(c) (5Z,13E)-(9α,15S)-N,N-Dimethyl-9,15-dihydroxy-11-oxo-15-(3-propylcyclopentyl)-16,17,18,19,20-pentanorprosta-5,13-dien-1-amide [i.e. 15-(3-Propylcyclopentyl)-16,17,18,19,20-pentanor-$PGD_2$ N,N-dimethylamide]

Starting material: 9,15-bis(tetrahydropyran-2-yloxy) compound prepared in Reference Example 12(c);

TLC(chloroform:tetrahydrofuran:acetic acid = 10:2:1): Rf = 0.18;

NMR: δ = 5.70-5.40(4H, m), 4.47(1H, m), 4.10(1H, m), 3.03(3H, s), 2.96(3H, s), 0.88(3H, t);

IR : ν = 3400, 2930, 1736, 1625 $cm^{-1}$;

Mass: m/e = 419($M^+$), 401.

(d) (5Z,13E)-(9α,15S)-N,N-Dimethyl-9,15-dihydroxy-11-oxo-16-phenyl-17,18,19,20-tetranorprosta-5,13-dien-1-amide (i.e. 16-Phenyl-17,18,19,20-tetranor-$PGD_2$ N,N-dimethylamide)

Starting material: 9,15-bis(tetrahydropyran-2-yloxy) compound prepared in Reference Example 12(d);

TLC(chloroform:tetrahydrofuran:acetic acid = 10:2:1): Rf = 0.16;

NMR: δ = 7.5-7.1(5H, m), 5.7-5.4(4H, m), 4.50(1H, m), 4.16(1H, m), 3.01(3H, s), 2.95(3H, s);

IR : ν = 3400, 2930, 1735, 1625 $cm^{-1}$;

Mass m/e = 399($M^+$), 381, 363.

(e) (5Z,13E)-(9α,15R)-N,N-Dimethyl-9,15-dihydroxy-11-oxo-16-(3-chlorophenoxy)-17,18,19,20-tetranorprosta-5,13-dien-1-amide [i.e. 16-(3-Chlorophenoxy)-17,18,19,20-tetranor-$PGD_2$ N,N-dimethylamide]

Starting material: 9,15-bis(tetrahydropyran-2-yloxy) compound prepared in Reference Example 12(e);

TLC(chloroform:tetrahydrofuran:acetic acid = 10:2:1): Rf = 0.16;

NMR: $\delta$ = 7.3-6.7(4H, m), 5.7-5.3(4H, m), 4.6-4.3(2H, m), 3.95(2H, d), 3.02(3H, s), 2.98(3H, s);

IR : $\nu$ = 3400, 2930, 1735, 1625, 1600 $cm^{-1}$;

Mass: m/e = 449($M^+$), 431, 413.

Example 7

(5Z,9Z,13E)-(15S)-N,N-Dimethyl-11-oxo-15-hydroxyprosta-5,9,13-trien-1-amide (i.e. 9-Deoxy-$\Delta^9$-$PGD_2$ N,N-dimethylamide)

To a solution of 57 mg of $PGD_2$ compound (prepared in Example 6) in 0.7 ml of ethanol, 70 ml of Tris-hydrochloric acid buffer solution (prepared by the same procedure described in Example 2) (pH 7.2) was added, and the resulting solution was stirred for 20 hours at 37°C. The reaction mixture was extracted with ethyl acetate. The extract was washed with a saturated aqueous solution of sodium chloride, dried, and concentrated under reduced pressure. The residue was purified by column chromatography on silica gel using a mixed solvent of n-hexane and ethyl acetate firstly and ethyl acetate finally as an eluent to give 14 mg of the title compound having the following physical characteristics:

TLC(chloroform:tetrahydrofuran:acetic acid = 10:2:1): Rf = 0.49;

NMR: $\delta$ = 7.59(1H, dd), 6.16(1H, dd), 5.7-5.3(4H, m), 4.18-4.02(1H, m), 2.99(3H, s), 2.94(3H, s), 3.0-2.8(1H, m), 2.72-2.64(1H, m), 1.0-0.8(3H, m);

IR : ν = 3420, 2930, 1710, 1630, 1400, 970 $cm^{-1}$;

Mass: m/e = 361($M^+$), 343.

The following compounds were obtained by the same procedure as described in Example 7:

(a) (9Z,13E)-(15S)-N,N-Dimethyl-11-oxo-15-hydroxyprosta-9,13-dien-1-amide (i.e. 9-Deoxy-$\Delta^9$-$PGD_1$ N,N-dimethylamide)

Starting material: $PGD_1$ compound prepared in Example 6(a);

TLC(chloroform:tetrahydrofuran:acetic acid = 10:2:1):

Rf = 0.49;

NMR: δ = 7.60(1H, dd), 6.15(1H, dd), 5.7-5.5(2H, m), 4.10(1H, m), 3.00(3H, s), 2.96(3H, s), 0.88(3H, t);

IR : ν = 3420, 2930, 1710, 1632 $cm^{-1}$;

Mass: m/e = 363($M^+$), 345.

(b) (5Z,9Z,13E)-(15S,17S)-N,N-Dimethyl-11-oxo-15-hydroxy-17,20-dimethylprosta-5,9,13-trien-1-amide (i.e. 17S,20-Dimethyl-9-deoxy-$\Delta^9$-$PGD_2$ N,N-dimethylamide)

Starting material: $PGD_2$ compound prepared in Example 6(b);

TLC(chloroform:tetrahydrofuran:acetic acid = 10:2:1):

Rf = 0.50;

NMR: δ = 7.58(1H, dd), 6.15(1H, dd), 5.7-5.3(4H, m), 4.10(1H, m), 3.00(3H, s), 2.95(3H, s), 1.0-0.8(6H, m);

IR : ν = 3430, 2930, 1710, 1630 $cm^{-1}$;

Mass: m/e = 389($M^+$), 371.

(c) (5Z,9Z,13E)-(15S)-N,N-Dimethyl-11-oxo-15-hydroxy-15-(3-propylcyclopentyl)-16,17,18,19,20-pentanorprosta-5,9,13-trien-1-amide [i.e. 15-(3-Propylcyclopentyl)-16,17,18,19,20-pentanor-9-deoxy-$\Delta^9$-$PGD_2$ N,N-dimethylamide]

Starting material: $PGD_2$ compound prepared in Example 6(c);

TLC(chloroform:tetrahydrofuran:acetic acid = 10:2:1):

Rf = 0.50;

NMR: δ = 7.60(1H, dd), 6.14(1H, dd), 5.7-5.3(4H, m), 4.10(1H, m), 3.00(3H, s), 2.94(3H, s), 0.88(3H, t);

IR : ν = 3410, 2930, 1713, 1632 $cm^{-1}$;

Mass: m/e = 401($M^+$), 383.

(d) (5Z,9Z,13E)-(15S)-N,N-Dimethyl-11-oxo-15-hydroxy-16-phenyl-17,18,19,20-tetranorprosta-5,9,13-trien-1-amide (i.e. 16-Phenyl-17,18,19,20-tetranor-9-deoxy-$\Delta^9$-$PGD_2$ N,N-dimethylamide)

Starting material: $PGD_2$ compound prepared in Example 6(d);

TLC(chloroform:tetrahydrofuran:acetic acid = 10:2:1):

Rf = 0.48;

NMR: δ = 7.60(1H, dd), 7.5-7.1(5H, m), 6.18(1H, dd), 5.7-5.3(4H, m), 3.00(3H, s), 2.95(3H, s);

IR : ν = 3420, 2930, 1715, 1632 $cm^{-1}$;

Mass: m/e = 381($M^+$), 363.

(e) (5Z,9Z,13E)-(15R)-N,N-Dimethyl-11-oxo-15-hydroxy-16-(3-chlorophenoxy)-17,18,19,20-tetranorprosta-5,9,13-trien-1-amide [i.e. 16-(3-Chlorophenoxy)-17,18,19,20-tetranor-9-deoxy-$\Delta^9$-$PGD_2$ N,N-dimethylamide]

Starting material: $PGD_2$ compound prepared in Example 6(e);

TLC(chloroform:tetrahydrofuran:acetic acid = 10:2:1):

Rf = 0.47;

NMR: δ = 7.65(1H, dd), 7.3-6.7(4H, m), 6.20(1H, dd), 5.74-5.30(4H, m), 3.00(3H, s), 2.97(3H, s);

IR : ν = 3430, 2930, 1710, 1636, 1600 $cm^{-1}$;

Mass: m/e = 431($M^+$), 413.

Example 8

(5Z,9Z,14EZ)-N,N-Dimethyl-11-oxoprosta-5,9,12,14-tetraen-1-amide

To a solution of 25 mg of $PGD_2$ compound (prepared in Example 6) in 0.5 ml of tetrahydrofuran, 0.5 ml of 1N aqueous hydrochloric acid was added, and the resulting solution was refluxed for 40 minutes. After cooling, the reaction mixture was extracted with diethyl ether, and the extract was washed with a saturated aqueous solution of sodium chloride, dried, and concentrated under reduced pressure. The residue was purified by column chromatography on silica gel using a mixed solvent of n-hexane and ethyl acetate as an eluent to give 14 mg of the title compound having the following physical characteristics:

TLC(ethyl acetate): Rf = 0.33;

NMR: $\delta$ = 7.54-7.44(1H, m), 6.95(1H, bd), 6.5-6.2(3H, m), 5.60-5.28(2H, m), 3.66-3.52(1H, m), 2.99(3H, s), 2.94(3H, s), 1.0-0.8(3H, m);

IR : $\nu$ = 2930, 1695, 1630, 1400, 1205, 1140, 980 $cm^{-1}$;

Mass: m/e = 343($M^+$), 272, 246.

The following compounds were obtained by the same procedure as described in Example 8:

(a) (9Z,14EZ)-N,N-Dimethyl-11-oxoprosta-9,12,14-trien-1-amide

Starting material: $PGD_1$ compound prepared in Example 6(a);

TLC(ethyl acetate): Rf = 0.34;

NMR: $\delta$ = 7.52-7.45(1H, m), 6.95(1H, d), 6.5-6.1(3H, m), 3,00(3H, s), 2.94(3H, s), 0.90(3H, t);

IR : $\nu$ = 2940, 1698, 1636 $cm^{-1}$;

Mass: m/e = 345($M^+$).

(b) (5Z,9Z,14EZ)-(17S)-N,N-Dimethyl-11-oxo-17,20-dimethylprosta-5,9,12,14-tetraen-1-amide

Starting material: $PGD_2$ compound prepared in Example 6(b);

TLC(ethyl acetate): Rf = 0.35;

NMR: δ = 7.50(1H, m), 6.95(1H, d), 6.5-6.2(3H, m), 5.6-5.3(2H, m), 3.6(1H, m), 3.00(3H, s), 2.94(3H, s), 1.0-0.8(6H, m);

IR : ν = 2930, 1698, 1635 $cm^{-1}$;

Mass: m/e = 371($M^+$).

(c) (5Z,9Z,14EZ)-N,N-Dimethyl-11-oxo-15-(3-propylcyclopentyl)-16,17,18,19,20-pentanorprosta-5,9,12,14-tetraen-1-amide

Starting material: $PGD_2$ compound prepared in Example 6(c);

TLC(ethyl acetate): Rf = 0.34;

NMR: δ = 7.54-7.44(1H, m), 6.95(1H, d), 6.5-6.14(3H, m), 5.6-5.26(2H, m), 3.5(1H, m), 3.00(3H, s), 2.94(3H, s), 0.90(3H, t);

IR : ν = 2930, 1700, 1633 $cm^{-1}$;

Mass: m/e = 383($M^+$).

(d) (5Z,9Z,14EZ)-N,N-Dimethyl-11-oxo-16-phenyl-17,18,19,20-tetranorprosta-5,9,12,14-tetraen-1-amide

Starting material: $PGD_2$ compound prepared in Example 6(d);

TLC(ethyl acetate): Rf = 0.32;

NMR: δ = 7.56-7.1(6H, m), 6.95(1H, d), 6.5-6.1(3H, m), 5.6-5.25(2H, m), 3.00(3H, s), 2.94(3H, s);

IR : ν = 2930, 1697, 1633 $cm^{-1}$;

Mass: m/e = 363($M^+$).

(e) (5Z,9Z,14EZ)-N,N-Dimethyl-11-oxo-16-(3-chlorophenoxy)-17,18,19,20-tetranorprosta-5,9,12,14-trien-1-amide

Starting material: $PGD_2$ compound prepared in Example 6(e);

TLC(ethyl acetate): Rf = 0.31;

NMR: δ = 7.54-6.70(6H, m), 6.5-6.1(3H, m), 5.5-5.3(2H, m), 3.02(3H, s), 2.98(3H, s);

IR : ν = 2930, 1700, 1632, 1600 $cm^{-1}$;

Mass: m/e = 413($M^+$).

Reference Example 13

(5Z,13E)-(9α,11α,15S)-9,15-Bis(tetrahydropyran-2-yloxy)-11-hydroxyprosta-5,13-dienoic acid methyl ester

To a solution of 1.08 g of 11-benzoyloxy compound (prepared in Reference Example 3) in 10 ml of methanol, 233 mg of potassium carbonate was added, and the resulting mixture was stirred for 2.5 hours at 50°C. To the reaction mixture, 50 ml of ethyl acetate was added, and the resulting mixture was washed with a saturated aqueous solution of sodium chloride, dried over anhydrous magnesium sulfate, and concentrated under reduced pressure. The residue was purified by column chromatography on silica gel using a mixed solvent of n-hexane and ethyl acetate as an eluent to give 681 mg of the title compound having the following physical characteristics:

TLC(cyclohexane:ethyl acetate = 2:1): Rf = 0.19;

NMR: δ = 5.7-5.1(4H, m), 4.8-4.4(2H, m), 3.6(3H, s), 0.9(3H, t);

IR : ν = 3470, 2930, 1737, 1124, 1018 $cm^{-1}$.

Reference Example 14

(5Z,13E)-(9α,11α,15S)-9,15-Bis(tetrahydropyran-2-yloxy)-11-hydroxyprosta-5,13-dien-1-amide

A solution of 3.6 g of ammonium chloride in 28 ml of concentrated aqueous ammonia was added to a solution of 637 mg of methyl ester compound (prepared in Reference Example 13) in 35 ml of ethanol, and the resulting mixture was allowed to react for 63 hours at 60°C in a sealed tube and concentrated under reduced pressure. After addition of water, the mixture was extracted with ethyl acetate. The extract was washed with a saturated aqueous solution of sodium chloride, dried over anhydrous magnesium sulfate, and concentrated under reduced pressure. The residue was purified by column chromatography on silica gel using ethyl acetate firstly and a mixed solvent of ethyl acetate and ethanol finally as an eluent to give 480 mg of the title compound having the following physical characteristics:

TLC(cyclohexane:ethyl acetate = 1:2): Rf = 0.10;

NMR: $\delta$ = 5.7(2H, bs), 5.6-5.2(4H, m), 4.8-4.4(2H, m), 4.3-3.1(7H, m), 0.9(3H, t);

IR : $\nu$ = 3400, 2930, 1670, 1020 $cm^{-1}$;

Mass: m/e = 436, 419, 401, 317.

Reference Example 15

(5Z,13E)-(9α,15S)-9,15-Bis(tetrahydropyran-2-yloxy)-11-oxo-prosta-5,13-dien-1-amide

At a temperature of -30 to -25°C, 322 μl of Jones' reagent (prepared by the same procedure described in Reference Example 9) was added dropwise to a solution of 373 mg of 11-hydroxy compound (prepared in Reference Example 14) in 3.73 ml of acetone. The resulting solution was stirred for

10 minutes at the same temperature, and 246 μl of isopropanol was added to the reaction mixture at that temperature. After warming to 0°C, 3 ml of water was added to the resulting mixture, and the mixture was extracted with ethyl acetate. The extract was washed with water and a saturated aqueous solution of sodium chloride, dried over anhydrous magnesium sulfate, and concentrated under reduced pressure. The residue was purified by column chromatography on silica gel using ethyl acetate as an eluent to give 203 mg of the title compound having the following physical characteristics:

TLC(cyclohexane:ethyl acetate = 1:5): Rf = 0.43;

NMR: δ = 6.0-5.0(6H, m), 4.8-4.3(2H, m), 4.3-4.2(6H, m), 0.9(3H, t);

IR : ν = 3350, 2930, 1743, 1670, 1130, 1022 $cm^{-1}$.

Example 9

(5Z,13E)-(9α,15S)-9,15-Dihydroxy-11-oxoprosta-5,13-dien-1-amide (i.e. $PGD_2$ amide)

To a solution of 231 mg of 9,15-bis(tetrahydropyran-2-yloxy) compound (prepared in Reference Example 15) in 0.5 ml of tetrahydrofuran, 5 ml of 65% (v/v) aqueous acetic acid was added, and the resulting mixture was stirred for 6 minutes at 70°C. After cooling, the reaction mixture was diluted with ethyl acetate, washed with water and a saturated aqueous solution of sodium chloride, dried over anhydrous magnesium sulfate, and concentrated with addition of toluene under reduced pressure at low temperature. The residue was purified by column chromatography on silica gel using ethyl acetate

firstly and a mixed solvent of ethyl acetate and ethanol finally as an eluent and by recrystallization from a mixed solution of ethyl acetate and n-hexane to give 131 mg of the title compound having the following physical characteristics:

Melting point : 71-73°C;

TLC(chloroform:tetrahydrofuran:acetic acid = 5:2:1): Rf = 0.34;

NMR: δ = 6.13(2H, s), 5.74-5.20(4H, m), 4.45(1H, m), 4.08(1H, m), 3.25(2H, s), 0.88(3H, t);

IR(KBr method): ν = 3430, 2930, 1727, 1659, 1400 $cm^{-1}$;

Mass: m/e = 333, 315, 280, 244.

Example 10

(5Z,9Z,13E)-(15S)-11-Oxo-15-hydroxyprosta-5,9,13-trien-1-amide (i.e. 9-Deoxy-$\Delta^9$-$PGD_2$ amide)

To a solution of 57 mg of $PGD_2$ compound (prepared in Example 9) in 0.7 ml of ethanol, 70 ml of Tris-hydrochloric acid buffer solution (prepared by the same procedure described in Example 2) (pH 7.2) was added, and the resulting mixture was stirred for 30 hours at 37°C. The reaction mixture was extracted with ethyl acetate. The extract was washed with a saturated aqueous solution of sodium chloride, dried over anhydrous magnesium sulfate, and concentrated under reduced pressure. The residue was purified by column chromatography on silica gel using a mixed solvent of ethyl acetate and n-hexane as an eluent to give 16 mg of the title compound having the following physical characteristics:

NMR: δ = 7.61(1H, dd), 6.16(1H, dd), 5.72-5.38(4H, m), 4.13(1H, m), 0.88(3H, t);

IR : $\nu$ = 3430, 2920, 1705, 1658, 1580 $cm^{-1}$;

Mass: m/e = 333($M^+$), 315.

Example 11

(5Z,9Z,14EZ)-11-Oxoprosta-5,9,12,14-tetraen-1-amide

To a solution of 25 mg of $PGD_2$ compound (prepared in Example 9) in 0.5 ml of tetrahydrofuran, 0.5 ml of a 1N aqueous hydrochloric acid was added, and the resulting solution was refluxed for 40 minutes. After cooling, the reaction mixture was extracted with diethyl ether. The extract was washed with a saturated aqueous solution of sodium chloride, dried over anhydrous magnesium sulfate, and concentrated under reduced pressure. The residue was purified by column chromatography on silica gel using a mixed solvent of ethyl acetate and n-hexane as an eluent to give 16 mg of the title compound having the following physical characteristics:

NMR: $\delta$ = 7.47(1H, m), 6.96(1H, d), 6.45-6.15(3H, m), 5.55-5.28(2H, m), 3.60(1H, m), 0.90(3H, t);

IR : $\nu$ = 2920, 1700, 1658, 1625 $cm^{-1}$;

Mass: m/e = 315($M^+$).

The present invention includes within its scope pharmaceutical compositions which comprise at least one prostaglandin D analogue of general formula (I) or cyclodextrin clathrate thereof, in association with a pharmaceutical carrier or coating.

In clinical practice, for the prevention or therapy (including therapy to secure remission) against leukemia or solid cancer, the compounds of the present invention will normally be administered systemically or partially; usually by oral or parenteral (e.g. intravenous, subcutaneous or intramuscular) administration.

Solid compositions for oral administration include compressed tablets, pills, dispersible powders and granules. In such solid compositions, one or more of the active compound(s) is, or are, admixed, with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents, e.g. lubricating agents such as magnesium stearate, and disintegrating agents, such as cellulose calcium gluconate. The tables or pills may, if desired be made into enteric film-coated or gastric film-coated tablets or pills, such as sugar-coated, gelatin-coated, hydroxypropylcellulose-coated or hydroxypropylmethylcellulose phthalate-coated tablets or pills; two or more layers may be used.

The compositions for oral administration also include capsules of absorbable material such as gelatin,

containing one or more of the active substances with or without the addition of diluents or excipients.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise at least one compound of the present invention.

Preparations according to the invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, and Polysorbate 80 (registered Trade Mark). These compositions may also include adjuvants such as preserving, wetting, emulsifying and dispersing agents. They may be sterilized, for example, by filtration

through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration. Such compositions are prepared by known methods.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when required for administration by injection; for oral administration the preparations will normally contain at least 0.1% by weight of active substance.

The dose to be administered is determined depending upon, for example, age, body weight, symptoms, the desired therapeutic effect , the route of administration, and the duration of the treatment.

In the human adult, the doses per person are

generally between 5 mg and 500 mg by oral administration, and between 500 µg and 50 mg by parenteral, preferably intravenous, administration for the prevention or the therapy (including therapy to secure remission) against leukemia or solid cancer, and can be administered up to several times per day.

As mentioned above, the doses to be used depend on various conditions. Therefore, there may be cases in which doses greater than the ranges specified above, or lower than the ranges specified above, may be used.

The following Examples illustrate pharmaceutical compositions according to the invention.

Example 12

A solution of 1 g of (5Z,9Z,14EZ)-1-hydroxymethyl-prosta-5,9,12,14-tetraene-1,11-dione in 5 ml of ethanol and 5 g of microcrystalline cellulose were well mixed and dried sufficiently. To the resulting mixture, 100 mg of magnesium stearate, 20 mg of silicon dioxide, 10 mg of talc and 200 mg of cellulose calcium gluconate (CCG) were admixed and then microcrystalline cellulose was added to make the total weight 10 g. The resulting mixture was well mixed to make it uniform, and then tabletted in conventional manner to give 100 tablets each containing 10 mg of the active material.

Example 13

α- and β-Cyclodextrin clathrate of (5Z,9Z,14EZ)-1-hydroxymethylprosta-5,9,12,14-tetraene-1,11-dione [prepared by the following procedure; To a solution of 2.4 g of α-cyclodextrin and 1 g of β-cyclodextrin in 300 ml of water, 100 mg of (5Z,9Z,14EZ)-hydroxymethylprosta-5,9,12,14-tetraene-1,11-dione was added, and the mixture was well stirred. The resulting solution was concentrated under reduced pressure.] were dissolved in 150 ml of distilled water for injection. The aqueous solution was filtered to sterilize it, pipetted into 5 ml ampoules to give 1.5 ml per ampoule, and lyophilized in conventional manner to give 100 ampoules for injection each containing 1 mg of the active material.

**CLAIMS** FOR CONTRACTING STATES: BE CH DE FR GB IT LI LU NL SE

1. Prostaglandin D analogues of the general formula:

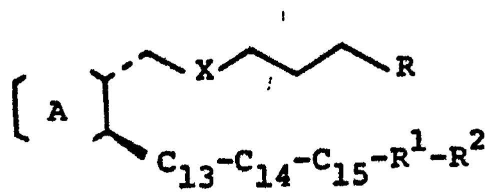

(I)

[wherein [A] represents a group of the formula:

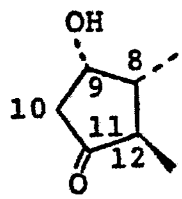

,

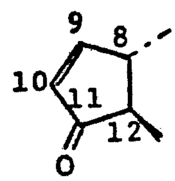

or

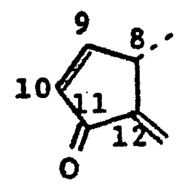

(II) (III) (IV)

X represents an ethylene group or a cis-vinylene group, $C_{13}-C_{14}-C_{15}$ represents

(i) a group of the formula:

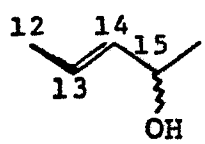

(V)

when [A] represents a group of the formula (II) or (III), or

(ii) a group of the formula:

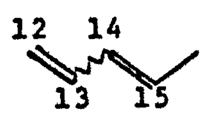

(VI)

when [A] represents a group of the formula (IV), R represents

(i) a group of the formula:

$-COCH_2OH$ or 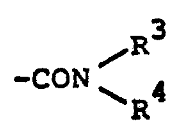

(wherein $R^3$ and $R^4$, which may be the same or different, each

represent a hydrogen atom or a straight-chain or branched-chain alkyl group of 1 - 4 carbon atoms) when [A] represents a group of the formula (II), or

(ii) a group of the formula:

$$-COCH_2OH\ ,\quad -CON\begin{matrix}R^3\\R^4\end{matrix}\quad \text{or}\quad -CONHSO_2R^5$$

(wherein $R^3$ and $R^4$ are as hereinbefore defined and $R^5$ represents a straight-chain or branched-chain alkyl group of 1 - 4 carbon atoms) when [A] represents a group of the formula (III) or (IV), $R^1$ represents a single bond or a straight-chain or branched-chain alkylene group of 1 - 5 carbon atoms, $R^2$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms, or a phenyl or phenoxy group either unsubstituted or substituted by at least one halogen atom, trifluoromethyl group or straight-chain or branched-chain alkyl group of 1 - 4 carbon atoms, the double bond between $C_9$ - $C_{10}$ in formula (III) and (IV) is Z, the double bond between $C_{13}$ - $C_{14}$ in formula (V) is E, and the double bonds between $C_{12}$ - $C_{13}$ and between $C_{14}$ - $C_{15}$ in formula (VI), which may be in the same or different configurations are E,Z or a mixture thereof, provided that when $R^1$ represents a single bond, $R^2$ does not represent a substituted or unsubstituted phenoxy group], and cyclodextrin clathrates thereof.

2. A prostaglandin analogue according to claim 1 wherein in general formula (V) the hydroxyl group attached to the carbon atom at the 15-position is in the α-configuration.

3. A prostaglandin analogue according to claim 1 or 2 wherein the grouping $-R^1-R^2$ represents a pentyl or hexyl group unsubstituted or substituted by one or two methyl groups; a cyclobutyl, cyclopentyl or cyclohexyl group unsubstituted or substituted by one methyl, ethyl, propyl or butyl group; or a phenyl or phenoxy group either unsubstituted or substituted by one chlorine atom, trifluoromethyl group, or methyl or ethyl group.

4. A prostaglandin analogue according to claim 1 which is 2-decarboxy-2-glycoloyl-$PGD_2$, 2-decarboxy-2-glycoloyl-17S,20-dimethyl-$PGD_2$, 2-decarboxy-2-glycoloyl-15-(3-propylcyclopentyl)-16,17,18,19,20-pentanor-$PGD_2$, 2-decarboxy-2-glycoloyl-16-phenyl-17,18,19,20-tetranor-$PGD_2$, 2-decarboxy-2-glycoloyl-16-(3-chlorophenoxy)-17,18,19,20-tetranor-$PGD_2$, 2-decarboxy-2-glycoloyl-$PGD_1$, 2-decarboxy-2-glycoloyl-9-deoxy-$\Delta^9$-$PGD_2$, 2-decarboxy-2-glycoloyl-17S,20-dimethyl-9-deoxy-$\Delta^9$-$PGD_2$, 2-decarboxy-2-glycoloyl-15-(3-propylcyclopentyl)-16,17,18,19,20-pentanor-9-deoxy-$\Delta^9$-$PGD_2$, 2-decarboxy-2-glycoloyl-16-phenyl-17,18,19,20-tetranor-9-deoxy-$\Delta^9$-$PGD_2$, 2-decarboxy-2-glycoloyl-16-(3-chlorophenoxy)-17,18,19,20-tetranor-9-deoxy-$\Delta^9$-$PGD_2$, 2-decarboxy-2-glycoloyl-9-deoxy-$\Delta^9$-$PGD_1$, (5Z,9Z,14EZ)-1-hydroxymethylprosta-5,9,12,14-tetraene-1,11-dione, (5Z,9Z,14EZ)-(17S)-1-hydroxymethyl-17,20-dimethylprosta-5,9,12,14-tetraene-1,11-dione, (5Z,9Z,14EZ)-1-hydroxymethyl-15-(3-propylcyclopentyl)-16,17,18,19,20-pentanor-prosta-5,9,12,14-tetraene-1,11-dione, (5Z,9Z,14EZ)-1-hydroxymethyl-16-phenyl-17,18,19,20-tetranorprosta-5,9,12,14-tetraene-1,11-dione, (5Z,9Z,14EZ)-1-hydroxymethyl-16-(3-chlorophenoxy)-17,18,19,20-tetranorprosta-5,9,12,14-tetraene-1,11-dione, (9Z,14EZ)-1-hydroxymethylprosta-9,12,14-triene-1,11-dione, $PGD_2$ amide, $PGD_2$ N,N-dimethylamide, 17S,20-dimethyl-$PGD_2$ N,N-dimethylamide, 15-(3-propylcyclopentyl)-16,17,18,19,20-pentanor-$PGD_2$ N,N-dimethylamide, 16-phenyl-17,18,19,20-tetranor-$PGD_2$ N,N-dimethylamide, 16-(3-chlorophenoxy)-17,18,19,20-tetranor-$PGD_2$ N,N-dimethylamide, $PGD_1$ N,N-dimethylamide, 9-deoxy-$\Delta^9$-$PGD_2$ amide, 9-deoxy-$\Delta^9$-$PGD_2$ N,N-dimethylamide, 17S,20-dimethyl-9-deoxy-$\Delta^9$-$PGD_2$ N,N-dimethylamide, 15-(3-propylcyclopentyl)-16,17,18,19,20-pentanor-9-deoxy-$\Delta^9$-$PGD_2$ N,N-dimethylamide, 16-phenyl-17,18,19,20-tetranor-9-deoxy-$\Delta^9$-$PGD_2$ N,N-dimethylamide, 16-(3-chlorophenoxy)-17,18,19,20-tetranor-9-deoxy-$\Delta^9$-$PGD_2$ N,N-dimethylamide, 9-deoxy-$\Delta^9$-$PGD_1$ N,N-dimethylamide, (5Z,9Z,14EZ)-11-oxoprosta-5,9,12,14-tetraen-1-amide, (5Z,9Z,14EZ)-N,N-dimethyl-11-oxoprosta-5,9,12,14-tetraen-1-amide, (5Z,9Z,14EZ)-(17S)-N,N-dimethyl-11-oxo-17,20-dimethylprosta-5,9,12,14-tetraen-1-amide, (5Z,9Z,14EZ)-N,N-dimethyl-11-oxo-15-(3-propylcyclopentyl)-16,17,18,19,20-pentanorprosta-5,9,12,14-tetraen-1-amide, (5Z,9Z,14EZ)-N,N-dimethyl-11-oxo-16-phenyl-17,18,19,20-tetranorprosta-5,9,12,14-tetraen-1-amide, (5Z,9Z,14EZ)-N,N-dimethyl-11-oxo-16-(3-chlorophenoxy)-17,18,19,20-tetranorprosta-5,9,12,14-tetraen-1-amide, (9Z,14EZ)-N,N-dimethyl-11-oxoprosta-9,12,14-trien-1-amide, 9-deoxy-$\Delta^9$-$PGD_2$ methanesulfonylamide, 17S,20-dimethyl-9-deoxy-$\Delta^9$-$PGD_2$ methanesulfonylamide, 15-(3-propylcyclopentyl)-16,17,18,19,20-pentanor-9-deoxy-$\Delta^9$-$PGD_2$ methanesulfonylamide, 16-phenyl-17,18,19,20-tetranor-9-deoxy-$\Delta^9$-$PGD_2$ methanesulfonylamide,

16-(3-chlorophenoxy)-17,18,19,20-tetranor-9-deoxy-$\Delta^9$-$PGD_2$ methanesulfonylamide, 9-deoxy-$\Delta^9$-$PGD_1$ methanesulfonylamide, (5Z,9Z,14EZ)-N-methanesulfonyl-11-oxoprosta-5,9,12,14-tetraen-1-amide, (5Z,9Z,14EZ)-(17S)-N-methanesulfonyl-11-oxo-17,20-dimethylprosta-5,9,12,14-tetraen-1-amide, (5Z,9Z,14EZ)-N-methanesulfonyl-11-oxo-15-(3-propylcyclopentyl)-16,17,18,19,20-pentanorprosta-5,9,12,14-tetraen-1-amide, (5Z,9Z,14EZ)-N-methanesulfonyl-11-oxo-16-phenyl-17,18,19,20-tetranorprosta-5,9,12,14-tetraen-1-amide, (5Z,9Z,14EZ)-N-methanesulfonyl-11-oxo-16-(3-chlorophenoxy)-17,18,19,20-tetranorprosta-5,9,12,14-tetraen-1-amide, or (9Z,14EZ)-N-methanesulfonyl-11-oxoprosta-9,12,14-trien-1-amide.

5. A cyclodextrin clathrate of a prostaglandin analogue claimed in claim 4.

6. A process for the preparation of prostaglandin D analogues as claimed in claim 1 which comprises (i) when [A] represents a group of formula (II), $C_{13}$-$C_{14}$-$C_{15}$ represents a group of formula (V), R represents a group of the formula $-COCH_2OH$ or $-CON\genfrac{}{}{0pt}{}{R^3}{R^4}$ wherein $R^3$ and $R^4$ are as defined in claim 1, and the other symbols are as defined in claim 1, the hydrolysis of a compound of the general formula:

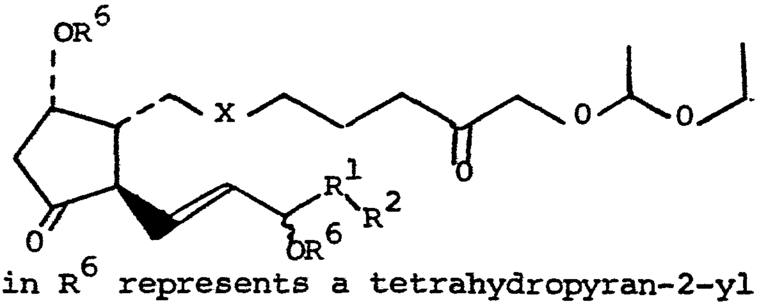

(XI)

(wherein $R^6$ represents a tetrahydropyran-2-yl group, a tetrahydrofuran-2-yl group either unsubstituted or substituted by at least one alkyl group or a 1-ethoxyethyl group and the other symbols are as defined in claim 1) or a compound of the general formula:

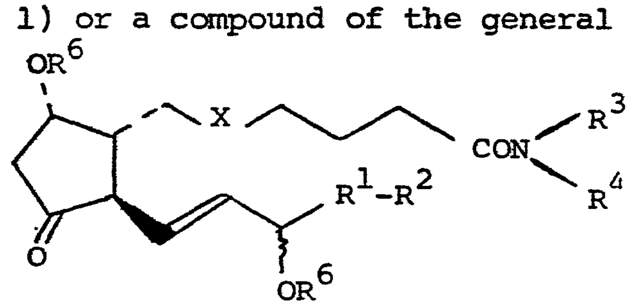

(XV)

(wherein $R^6$ is as hereinbefore defined and the other symbols are as defined in claim 1) under acidic conditions

(ii) when [A] represents a group of formula (III), $C_{13}-C_{14}-C_{15}$ represents a group of formula (V), R represents a group of the formula;

$-COCH_2OH$, 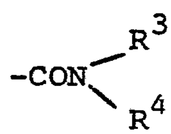 or $-CONHSO_2R^5$

wherein $R^3$, $R^4$ and $R^5$ are as defined in claim 1 and the other symbols are as defined in claim 1, the dehydration under mild conditions of a compound of the general formula:

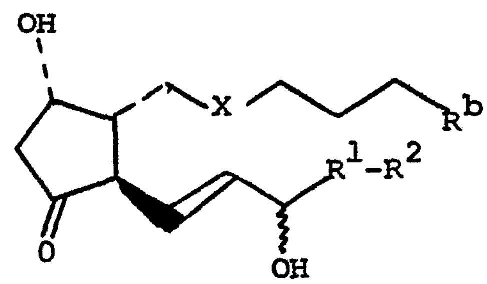

(VII)

wherein $R^b$ represents a group of the formula

$-COCH_2OH$ 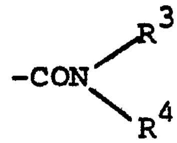 or $CONHSO_2R^5$

wherein $R^3$, $R^4$ and $R^5$ are as defined in claim 1, and the other symbols are as defined in claim 1,

(iii) when [A] represents a group of formula (III), $C_{13}-C_{14}-C_{15}$ represents a group of formula (V), R represents a group $R^b$ as hereinbefore defined and the other symbols are as defined in claim 1, the hydrolysis under acidic conditions of a compound of the general formula:

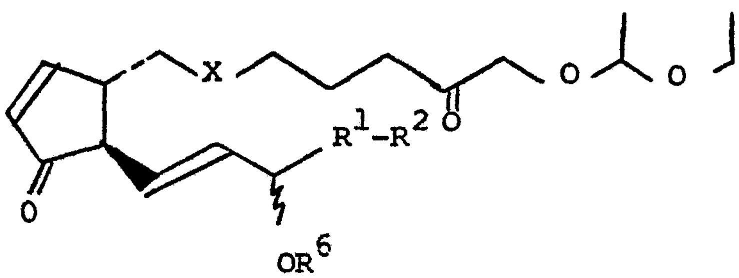

(XXX)

(wherein $R^6$ is as hereinbefore defined and the other symbols are as defined in claim 1 ) or of a compound of the general formula:

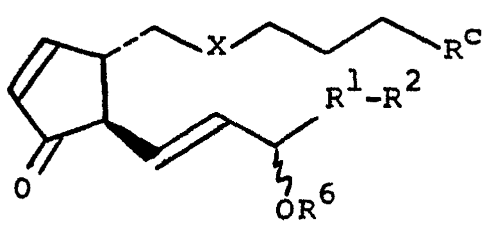

(XXXVI)

wherein $R^c$ represents a group of the formula:

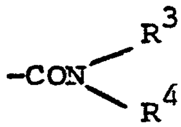 or $-CONHSO_2R^5$

(wherein $R^3$, $R^4$ and $R^5$ are as defined in claim 1), $R^6$ is as hereinbefore defined and the other symbols are as defined in claim 1, or

(iv) when [A] represents a group of formula (IV), $C_{13}-C_{14}-C_{15}$ represents a group of formula (VI), R represents a group $R^b$ as hereinbefore defined and the other symbols are as defined in claim 1, reacting with an aqueous acid solution a compound of the general formula (VII), (XI), (XV), (XXX) or (XXXVI), a compound of the general formula:

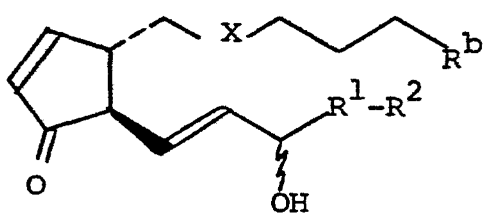

(Ib)

or a compound of the general formula:

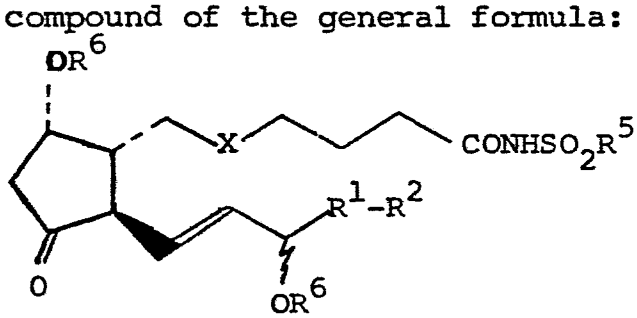

(XVII)

in which formulae (Ib), (VII), (XI), (XV), (XVII), (XXX) or (XXXVI), $R^6$ and $R^b$ are as hereinbefore defined, and the other symbols are as defined in claim 1, optionally followed by the step of converting a prostaglandin D analogue of general formula (I) into a cyclodextrin clathrate thereof.

7. A pharmaceutical composition which comprises, as active ingredient, a prostaglandin analogue of general formula (I) depicted in claim 1, wherein the various symbols are as defined in claim 1, or a cyclodextrin clathrate thereof, in association with a pharmaceutical carrier or coating.

8. A prostaglandin analogue, or cyclodextrin clathrate thereof, according to any one of claims 1 to 5 for use in the prevention or therapy of leukemia or solid cancer.

9. A compound of the general formula (XI), (XV), (XVII), (XXX) or (XXXVI) depicted in claim 6, wherein $R^6$ and $R^c$ are as defined in claim 6 and the other symbols are as defined in claim 1.

CLAIMS FOR CONTRACTING STATE: AT

1. A process for the preparation of prostaglandin D analogues of the general formula:

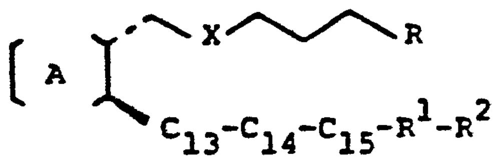

(I)

[wherein [A] represents a group of the formula:

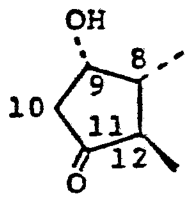 , 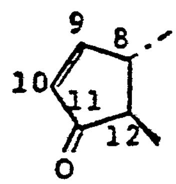 or 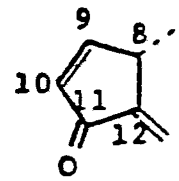

(II) (III) (IV)

X represents an ethylene group or a cis-vinylene group,

$C_{13}-C_{14}-C_{15}$ represents

(i) a group of the formula:

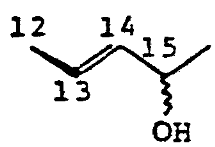

(V)

when [A] represents a group of the formula (II) or (III), or

(ii) a group of the formula:

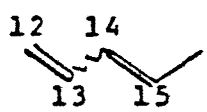

(VI)

when [A] represents a group of the formula (IV), R represents

(i) a group of the formula:

$-COCH_2OH$ or 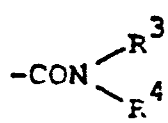

(wherein $R^3$ and $R^4$, which may be the same or different, each

represent a hydrogen atom or a straight-chain or branched-chain alkyl group of 1 - 4 carbon atoms) when [A] represents a group of the formula (II), or

(ii) a group of the formula:

$$-COCH_2OH \ , \quad -CON\begin{matrix} R^3 \\ R^4 \end{matrix} \quad \text{or} \quad -CONHSO_2R^5$$

(wherein $R^3$ and $R^4$ are as hereinbefore defined and $R^5$ represents a straight-chain or branched-chain alkyl group of 1 - 4 carbon atoms) when [A] represents a group of the formula (III) or (IV), $R^1$ represents a single bond or a straight-chain or branched-chain alkylene group of 1 - 5 carbon atoms, $R^2$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms, or a phenyl or phenoxy group either unsubstituted or substituted by at least one halogen atom, trifluoromethyl group or straight-chain or branched-chain alkyl group of 1 - 4 carbon atoms, the double bond between $C_9$ - $C_{10}$ in formula (III) and (IV) is Z, the double bond between $C_{13}$ - $C_{14}$ in formula (V) is E, and the double bonds between $C_{12}$ - $C_{13}$ and between $C_{14}$ - $C_{15}$ in formula (VI), which may be in the same or different configurations are E,Z or a mixture thereof, provided that when $R^1$ represents a single bond, $R^2$ does not represent a substituted or unsubstituted phenoxy group], and cyclodextrin clathrate thereof, which process comprises:

(i) when [A] represents a group of formula (II), $C_{13}-C_{14}-C_{15}$ represents a group of formula (V), R represents a group of the formula $-COCH_2OH$ or

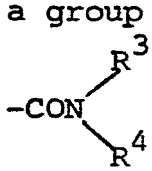 wherein $R^3$ and $R^4$ are as hereinbefore defined

and the other symbols are as hereinbefore defined, the hydrolysis of a compound of the general formula:

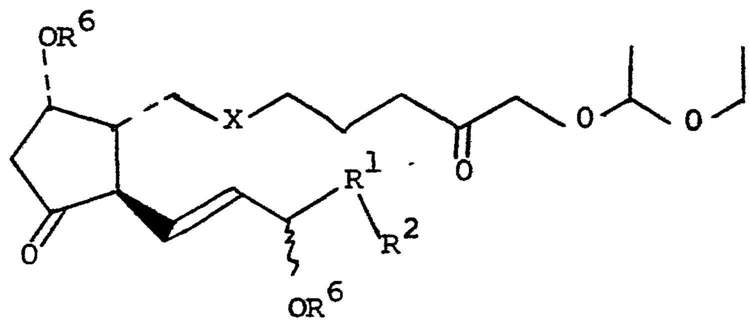

(XI)

(wherein $R^6$ represents a tetrahydropyran-2-yl group, a tetrahydrofuran-2-yl group either unsubstituted or substituted by at least one alkyl group or a 1-ethoxyethyl group and the other symbols are as hereinbefore defined) or a compound of the general formula:

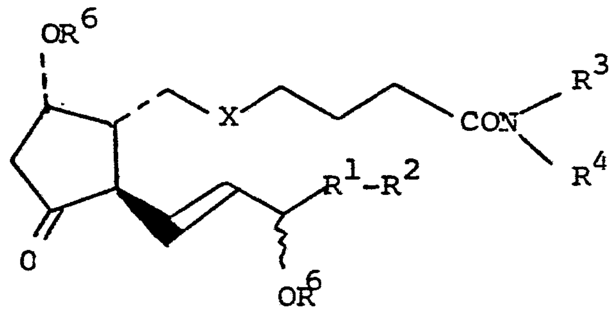

(XV)

(wherein the various symbols are as hereinbefore defined) under acidic conditions,

(ii) when [A] represents a group of formula (III), $C_{13}-C_{14}-C_{15}$ represents a group of formula (V), R represents a group of the formula $-COCH_2OH$, 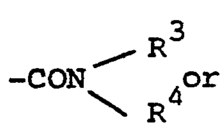 or $-CONHSO_2R^5$

wherein $R^3$, $R^4$ and $R^5$ are as hereinbefore defined and the

other symbols are as hereinbefore defined, the dehydration under mild conditions of a compound of the general formula:

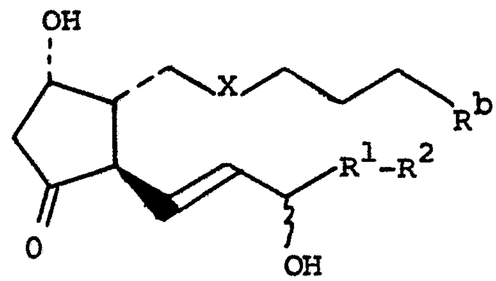

(VII)

wherein $R^b$ represents a group of the formula:

$-COCH_2OH$ 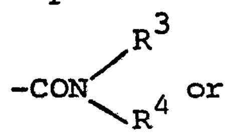 or $CONHSO_2R^5$

wherein $R^3$ and $R^4$ are as hereinbefore defined, and the other symbols are as hereinbefore defined,

(iii) when [A] represents a group of formula (III), $C_{13}-C_{14}-C_{15}$ represents a group of formula (V), R represents a group $R^b$ as hereinbefore defined, and the other symbols are as hereinbefore defined, the hydrolysis under acidic conditions of a compound of the general formula:

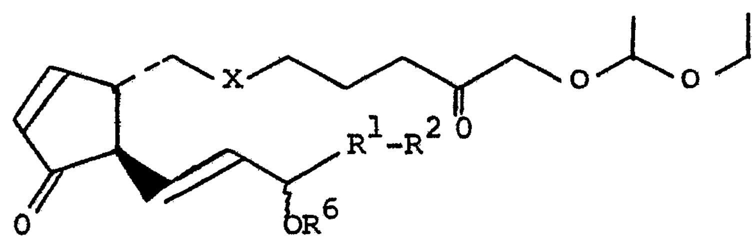

(XXX)

(wherein the various symbols are as hereinbefore defined) or of a compound of the general formula:

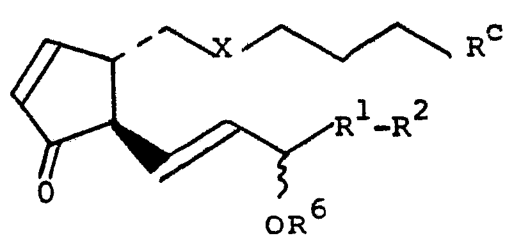

(XXXVI)

wherein $R^c$ represents a group of the formula:

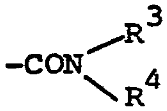 or $-CONHSO_2R^5$

wherein $R^3$, $R^4$ and $R^5$ are as hereinbefore defined and the other symbols are as hereinbefore defined, or

(iv) when [A] represents a group of formula (IV), $C_{13}-C_{14}-C_{15}$ represents a group of formula (VI), R represents a group $R^b$ as hereinbefore defined and the other symbols are as hereinbefore defined, reacting with an aqueous acid solution a compound of the general formula (VII), (XI), (XV), (XXX), or (XXXVI), a compound of the general formula:

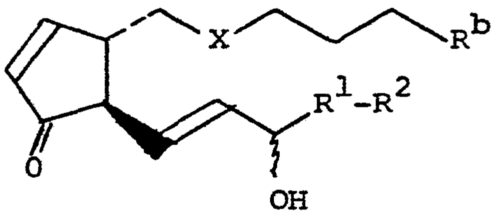

(Ib)

or a compound of the general formula:

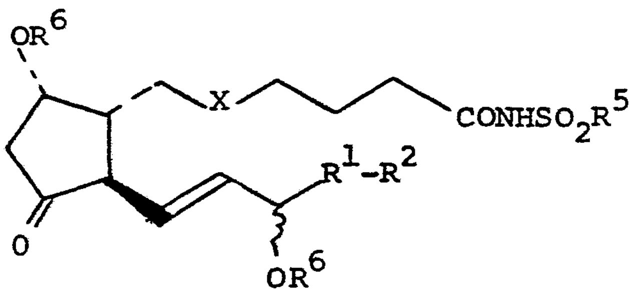

(XVII)

in which formulae (Ib), (VII), (XI), (XV), (XVII), (XXX) or (XXXVI), the various symbols are as hereinbefore defined, optionally followed by the step of converting a prostaglandin D analogue of general formula (I) into a cyclodextrin clathrate thereof.

2. A process according to claim 1 in which the symbol $R^6$ represents a tetrahydropyran-2-yl group and the other symbols are as defined in claim 1.

3. A process according to claim 1 or 2 in which the hydrolysis under acidic conditions of the compound of the general formula (XI) and (XV), wherein the various symbols are as defined in claim 1 or 2, is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulphonic acid and anhydrous methanol.

4. A process according to claim 1 in which the dehydration under mild conditions of the compound of the general formula (VII), wherein the various symbols are as defined in claim 1, is carried out by reaction with a tris-hydrochloric acid buffer solution at a temperature of 30-40°C.

5. A process according to claim 1 or 2 in which the hydrolysis under acidic conditions of the compound of the general formula (XXX) or (XXXVI), wherein the various symbols are as defined in claim 1 or 2, is carried out under the conditions specified in claim 3 for the hydrolysis of a compound of the general formula (XI) or (XV).

6. A process according to claim 1 or 2 in which the reaction with an aqueous acid solution of the compound of the general formula (Ib), (VII), (XI), (XV), (XVII), (XXX), or (XXXVI) is carried out in tetrahydrofuran using 1N hydrochloric acid at the reflux temperature of the reaction mixture.